# EUROPÄISCHE PATENTANMELDUNG

(11) **EP 4 303 184 A1**
(43) Veröffentlichungstag der Anmeldung: **10.01.2024**
(21) Anmeldenummer: 22183760.2
(22) Anmeldetag: 08.07.2022
(51) Int. Cl.: C01B 32/15, B82B 3/00, B82Y 30/00, B82Y 40/00, C05F 11/00, H01G 11/36

(54) **VERWENDUNGEN EINES AUS EINEM VERFAHREN ZUR STOFFLICHEN BEHANDLUNG VON ROHSTOFFEN HERGESTELLTEN KOHLENSTOFFS**

(71) Anmelder: Merenas Trust Reg., 9490 Vaduz (LI)
(72) Erfinder: Hemmerle, Karl-Heinz, FL-9490 Vaduz (LI)
(74) Vertreter: Löbl, Stefan

(57) **Zusammenfassung**

Die Erfindung betrifft Verwendungen eines aus einem auf einem Verschwelungs- und Destillationsprozess basierenden Verfahren zur stofflichen Behandlung von Rohstoffen hergestellten Kohlenstoffs mit einer Struktur einer dreidimensionalen Anordnung von Kohlenstoffnanoteilchen. Der Kohlenstoff dient dabei zur medizinischen Verwendung, als thermischer und/oder feuerfester und/oder strahlungsresistenter Isolierstoff, als ein Filterelement, als ein Speicherelement oder zum Herstellen von pflanzlichen Produkten oder zum Bepflanzen von wasserarmen Gebieten.

Die Erfindung betrifft zudem eine Speichervorrichtung (40) als elektrischer Energiespeicher in Form eines Doppelschicht-Kondensators mit einem symmetrischen Aufbau mit von außen nach innen jeweils einem Gehäuse (41) und einem Kollektor (42) mit einer als Kohlenstoffschicht ausgebildeten Elektrode (43) und einem Separator (44) mit einem Elektrolyten. Dabei ist die Kohlenstoffschicht aus einem mit dem Verfahren zur stofflichen Behandlung von Rohstoffen hergestellten Kohlenstoff mit der Struktur der dreidimensionalen Anordnung von Kohlenstoffnanoteilchen ausgebildet.

## Beschreibung

Die Erfindung betrifft Verwendungen eines aus einem auf einem Verschwelungs- und Destillationsprozess basierenden Verfahren zur stofflichen Behandlung von Rohstoffen hergestellten Kohlenstoffs.

Aus dem Stand der Technik bekannte Vorrichtungen und Verfahren sind für die industrielle Behandlung, insbesondere von Altgummiprodukten, Gummierzeugnissen oder gummiähnlichen Verbunderzeugnissen, wie Altreifen, stahlseilbewehrten Gummigurten, gummierten Kettengliedern und Förderbändern, sowie von zerkleinerten Altfahrzeugen, von organischen nachwachsenden Rohstoffen, wie Holz, von verseuchten Kohlenstoffen und verseuchten Böden vorgesehen. Dabei werden leichtes Öl, Gas, Metalle, insbesondere Stahl, und Kohlenstoff gewonnen. Herkömmliche Anlagen basieren beispielsweise auf der Verwendung von Drehrohröfen, Wirbelbettreaktoren sowie Trommeln und verarbeiten verdichtetes Ausgangsmaterial unter chemisch inerter Atmosphäre unter Ausschluss von Sauerstoff.

In der DE 199 30 071 C2 werden ein Verfahren und eine Vorrichtung zur Verwertung von organischen Stoffen und Stoffgemischen beschrieben. Das organische Material wird dabei mit Wirbelbettmaterial der Verbrennungswirbelschicht in Kontakt gebracht. Bei dem Verfahren entstehen Endprodukte in Form von Gasen mit kondensierbaren Substanzen und kohlenstoffhaltige Rückstände.

In der DE 39 32 803 A1 wird ein Reaktionsverfahren von organischen Materialien unter Zusatz von Borsäure/Boroxid und organischen Stickstoffverbindungen in nichtoxidierender Atmosphäre zu Kohle und Graphit offenbart.

Für das Betreiben herkömmlicher Anlagen sind erhöhte Aufwendungen für Material, Energie und Logistik erforderlich. So bedingt beispielsweise die Erzeugung einer Wirbelschicht bei Wirbelbettreaktoren einen erhöhten Energieaufwand, da einerseits die Wirbelschicht erzeugt und erhalten werden muss und andererseits die zu verwertenden Materialien mechanisch so aufgearbeitet werden müssen, dass sie mit der Wirbelschicht wirksam kontaktieren. Auch durch das Zerkleinern beziehungsweise Verdichten der Ausgangsstoffe in der Vorbereitung und während des Verwertungsvorgangs fallen hohe Energiekosten an.

In der WO 2007/053088 A1 werden ein Verfahren sowie eine Vorrichtung zur Behandlung von Materialien aus Kohlenwasserstoffen beschrieben. Die Materialien werden in einen inneren Behälter beschickt, welcher wiederum in einem äußeren Behälter anordenbar ist. Beide Behälter werden jeweils mit einem Deckelelement verschlossen. Das Kohlenwasserstoff-Material wird mittels Mikrowellen oder hochfrequenter Bestrahlung erwärmt. Die entstehenden Abgase werden durch einen Gasauslass aus den Behältern abgeführt. Es können zwei oder mehr Behälter parallel betrieben und an einer Gasreinigungsanlage angeschlossen werden, um einen nahezu kontinuierlichen Gasstrom durch die Gasreinigungsanlage aufrechtzuerhalten.

Die WO 2010/012275 A2 offenbart eine Vorrichtung zur Behandlung von Materialien mit einem zylinderförmigen Ofen sowie eine Steuerung des Prozesses. Die Innenflächen des Ofens sind mit einer Isolierschicht aus einem anorganischen Wärmedämmstoff versehen. Auf oder an den Innenflächen der Isolierschichten sind Heizelemente angeordnet. Die Steuerung des Prozesses über eine Regelung der Temperatur der Heizelemente dient einer hohen Ausbeute an Kohlenstoff, Öl und Brenngas.

In der DE 10 2012 109 874 A1 sind eine Vorrichtung zur stofflichen Behandlung von Rohstoffen mit einem Heizsystem, einer Destillationseinheit und einer mit den Rohstoffen beschickbaren Reaktionseinheit sowie ein Verfahren zum Betreiben einer solchen Vorrichtung gezeigt. Das Heizsystem, welches zur Bestückung mit der Reaktionseinheit geöffnet und geschlossen werden kann, weist ein Kopfelement und ein fest mit dem Kopfelement verbundenes Mantelelement sowie Stützelemente auf. Das Kopfelement ist mit den in vertikaler Richtung in der Länge veränderbaren Stützelementen derart verbunden, dass durch ein Verändern der Länge der Stützelemente zwischen zwei Endpositionen das Heizsystem in vertikaler Bewegungsrichtung geöffnet und geschlossen wird.

Aufgabe der vorliegenden Erfindung ist es, einen mit einem Verfahren zur stofflichen Behandlung von Rohstoffen, insbesondere unterschiedlichen Altgummiprodukten, wie Altreifen und Gummiverbunderzeugnisse oder gummiähnlichen Verbunderzeugnissen, nachwachsenden Rohstoffen, wie Holz, Schalen oder Früchten, Elektronikschrott, wie Computern und Mobiltelefonen, Kraftfahrzeugen und Speichermedien, wie Batterien, hergestellten Kohlenstoff mit von herkömmlichen Kohlenstoffen abweichenden, vorteilhaften stofflichen Eigenschaften für verschiedene Verwendungen bereitzustellen.

Die Lösung der Aufgabe der Erfindung besteht in Verwendungen von Kohlenstoff mit einer Struktur einer dreidimensionalen Anordnung von Kohlenstoffnanoteilchen, welcher durch ein Verfahren zur stofflichen Behandlung von Rohstoffen hergestellt wird. Das Verfahren weist folgende Schritte auf:
- Erwärmen einer mit Rohstoffen beschickten und in einem geschlossenen Heizsystem angeordneten Reaktionseinheit sowie Starten eines Verschwelungs- und Destillationsprozesses, wobei der Verschwelungs- und Destillationsprozess durch gezieltes Beheizen bei einer im Wesentlichen konstanten Temperatur innerhalb der Reaktionseinheit erfolgt,
- Abführen entstehender Gase aus der Reaktionseinheit in eine Destillationseinheit durch eine zwischen der Reaktionseinheit und der Destillationseinheit ausgebildete Abgasleitung sowie Bestimmen der Temperatur des durch die Abgasleitung hindurchströmenden Gases,
- Abkühlen und Kondensieren der Gase in der Destillationseinheit, wobei durch eine Zwangskühlung einer Abkühlstrecke der Destillationseinheit die Temperatur der Gase über eine von den Gasen abgeführte Wärmeleistung gesteuert wird, und
- Absaugen nichtkondensierbarer Gase, wobei innerhalb der Reaktionseinheit ein Unterdruck zur Umgebung erzeugt und Sauerstoff aus der Reaktionseinheit abgeführt wird.

Der Kohlenstoff dient erfindungsgemäß zur medizinischen Verwendung, als thermischer und/oder feuerfester Isolierstoff, als ein Filterelement, als ein Speicherelement oder zum Herstellen von pflanzlichen Produkten oder zum Bepflanzen von wasserarmen Gebieten.

Das Verfahren basiert vorteilhaft auf einem Betreiben einer Vorrichtung zur stofflichen Behandlung von Rohstoffen. Die Vorrichtung weist ein Heizsystem, eine Destillationseinheit und eine Reaktionseinheit sowie eine Steuervorrichtung auf. Die Reaktionseinheit ist mit den Rohstoffen beschickbar. Das Heizsystem ist zur Bestückung mit der Reaktionseinheit öffenbar und verschließbar. Die Destillationseinheit weist eine Abkühlstrecke auf.

Im Bereich des Heizsystems und der Destillationseinheit sind Temperatursensoren ausgebildet. Zudem weist die Abkühlstrecke der Destillationseinheit eine Vorrichtung zur Zwangskühlung auf. Die Vorrichtung zur Zwangskühlung der Abkühlstrecke ermöglicht, dass die Abkühlstrecke, im Vergleich beispielsweise zu freier Konvektion, gezielt mit einem Wärmeträgerfluid - insbesondere gasförmig oder flüssig - zum Abführen von Wärme angeströmt beziehungsweise umströmt wird.

Die Vorrichtung zur stofflichen Behandlung von Rohstoffen weist eine Absaugvorrichtung zum Absaugen von Gasen aus der Reaktionseinheit und Erzeugen eines Unterdrucks innerhalb der Reaktionseinheit auf. Der Unterdruck bezieht sich auf den Druck der Umgebung der Vorrichtung. Die Absaugvorrichtung kann als eine Pumpe, insbesondere als eine Membranpumpe ausgebildet sein.

Die Temperatursensoren und die Absaugvorrichtung sind mit der Steuervorrichtung verbunden.

Die Vorrichtung weist vorzugsweise mindestens zwei Temperatursensoren zum Bestimmen der Temperatur innerhalb der Reaktionseinheit auf, welche im geschlossenen Zustand des Heizsystems in einem zwischen der Reaktionseinheit und einem Mantelelement des Heizsystems ausgebildeten Zwischenraum angeordnet sind.

Eine zwischen dem Heizsystem und der Destillationseinheit ausgebildete Abgasleitung kann eine Heizvorrichtung zum Erwärmen der Abgasleitung aufweisen. Die vorzugsweise die Abgasleitung vollständig umschließende und vorteilhaft elektrisch betriebene Heizvorrichtung ist mit der Steuervorrichtung verbunden.

An der zwischen dem Heizsystem und der Destillationseinheit ausgebildeten Abgasleitung ist bevorzugt mindestens ein Temperatursensor zum Bestimmen der Temperatur von aus dem Heizsystem abgeführter Abgase vorgesehen.

An der zwischen dem Heizsystem und der Destillationseinheit ausgebildeten Abgasleitung kann ein Anschlusselement zum Verbinden mit einer Vorrichtung zum Einlassen eines gasförmigen Spülmediums, insbesondere in die Reaktionseinheit, vorgesehen sein. Das Spülmedium, beispielsweise Stickstoff, dient dem Inertisieren innerhalb der Reaktionseinheit, reduziert die Explosionsgefahr und unterstützt als Trägergas das Trennen von bei einem Betreiben der Vorrichtung entstehenden Endprodukten.

Die Abkühlstrecke der Destillationseinheit ist vorteilhaft innerhalb eines Luftleitgehäuses angeordnet. Dabei sind innerhalb einer Wandung des Luftleitgehäuses Lüfter zum gezielten Leiten von Umgebungsluft über die Abkühlstrecke vorgesehen. Das Luftleitgehäuse ist mit den Lüftern als Vorrichtung zur Zwangskühlung der Abkühlstrecke der Destillationseinheit mit Umgebungsluft ausgebildet. Die Lüfter sind mit der Steuervorrichtung verbunden.

Die innerhalb der Wandung des Luftleitgehäuses der Abkühlstrecke der Destillationseinheit ausgebildeten Lüfter zum gezielten Leiten von Umgebungsluft über die Abkühlstrecke sind vorzugsweise an einer Oberseite, insbesondere an einer in vertikaler Richtung nach oben weisenden Stirnfläche, oder an einer Seitenfläche des Luftleitgehäuses angeordnet.

Alternativ kann die Abkühlstrecke aus mindestens einem doppelwandigen Koaxialrohr als Vorrichtung zur Zwangskühlung der Abkühlstrecke zum Durchleiten von Gasen im Inneren eines innenliegenden Rohres und zum Durchleiten eines Wärmeträgerfluids im Zwischenraum zwischen der Außenseite des innenliegenden Rohres und der Innenseite des außenliegenden Rohres ausgebildet sein. Das Wärmeträgerfluid liegt vorzugsweise im flüssigen Aggregatszustand vor und ist insbesondere Wasser oder Glykol.

Ein Vorteil der Vorrichtung zur stofflichen Behandlung von Rohstoffen besteht darin, dass die Absaugvorrichtung zum Absaugen von Gasen aus der Reaktionseinheit und Erzeugen eines Unterdrucks innerhalb der Reaktionseinheit in Strömungsrichtung der Gase nach einem der Destillationseinheit nachgeordneten Öltank angeordnet ist. Damit wird der Unterdruck auch innerhalb der Destillationseinheit erzeugt.

Das Heizsystem kann ein Kopfelement und das fest mit dem Kopfelement verbunden ausgebildete Mantelelement sowie Stützelemente aufweisen. Das Kopfelement ist dabei an den in vertikaler Richtung in der Länge veränderbaren Stützelementen gehaltert angeordnet. Durch die Veränderung der Länge der Stützelemente zwischen zwei Endpositionen wird das Heizsystem in vertikaler Bewegungsrichtung geöffnet und geschlossen.

Dabei weist das Heizsystem vorzugsweise zwei Stützelemente auf, welche bevorzugt beiderseits des Heizsystems angeordnet sind. Nach einer ersten Alternative werden die Stützelemente mit Elektrospindeln angetrieben. Nach einer zweiten Alternative sind die Stützelemente als Hydraulikstützen ausgebildet.

Nach einer Weiterbildung der Vorrichtung ist das Mantelelement mit einer hohlzylinderförmigen Wandung ausgebildet. Die Wandung ist in vertikaler Richtung nach unten geöffnet und nach oben mit einer kreisrunden Haube verschlossen. Das Mantelelement ist an der Haube mit dem Kopfelement zu einer Einheit verbunden.

Das Mantelelement weist vorteilhaft gleichmäßig am Umfang der inneren Fläche der Wandung verteilt angeordnete Heizelemente auf. Die Wandung ist zur Verhinderung des Wärmeüberganges zur Umgebung nach außen mit einer Wärmeisolierung aus Keramikpulver ausgebildet.

Die Haube kann am Mittelpunkt mit einem Abgasstutzen als Verbindung zu einer Abgasleitung des Heizsystems ausgebildet sein. Die Abgasleitung erstreckt sich dabei vom Abgasstutzen durch die Haube hindurch in das Kopfelement des Heizsystems.

Die Abgasleitung weist am distalen Ende zum Abgasstutzen der Haube vorteilhaft ein Verbindungselement als Verbindung mit der Abgasleitung der Destillationseinheit auf.

Die sich vom Abgasstutzen durch die Haube hindurch in das Kopfelement des Heizsystems erstreckende Abgasleitung kann im Bereich des Abgasstutzens zur Kompensation thermischer Ausdehnungen mit einer, insbesondere in vertikaler Richtung, selbsttätig längenveränderlichen Rohrverbindung ausgebildet sein.

Ein weiterer Vorteil der Vorrichtung besteht darin, dass die Reaktionseinheit mit einer Wandung in Form eines hohlzylindrischen Gefäßes ausgebildet ist, welches am Boden verschlossen ist. Dabei ist die offene Seite der Wandung mittels eines Deckelelementes verschließbar.

Zwischen der Wandung und dem Deckelelement ist vorteilhaft eine hochtemperaturbeständige Dichtung angeordnet.

Das Deckelelement der Reaktionseinheit ist bevorzugt kreisrund ausgebildet und weist am Mittelpunkt den Abgasstutzen auf. Von besonderem Vorteil ist, dass der Abgasstutzen des Deckelelementes und der Abgasstutzen des Mantelelementes im geschlossenen Zustand des Heizsystems ineinandergreifen und eine dichte Verbindung zur Abgasleitung ausbilden.

Das Deckelelement der Reaktionseinheit kann mit einem Anschlussstutzen zum Verbinden mit einer Vorrichtung zum Einlassen eines gasförmigen Spülmediums, insbesondere von Stickstoff, in die Reaktionseinheit ausgebildet sein.

Die Reaktionseinheit kann im Inneren Siebelemente aufweisen, welche vorzugsweise horizontal ausgerichtet und in unterschiedlichen Höhen, beabstandet zueinander angeordnet sind. Die Siebelemente bedecken dabei bevorzugt den gesamten Querschnitt der Reaktionseinheit.

Die Steuervorrichtung der Vorrichtung zur stofflichen Behandlung von Rohstoffen dient zum Steuern eines Verfahrens zum Betreiben der Vorrichtung und ist dabei neben den Temperatursensoren, der Vorrichtung zur Zwangskühlung, insbesondere den Fördervorrichtungen, wie den Lüftern zum gezielten Leiten von Umgebungsluft über die Abkühlstrecke oder mindestens einer Pumpe zum Fördern des flüssigen Wärmeträgerfluids, und der Absaugvorrichtung vorteilhaft ebenso mit einem Antrieb der Stützelemente, einem Füllstandssensor des Öltanks, einem Drucksensor und Ventilen von Heizkreisen des Heizsystems verbunden. Der Füllstandssensor des Öltanks kann als Schwimmer ausgebildet sein. Der Drucksensor ist vorteilhaft im Bereich des Öltanks angeordnet. Dabei kann die Steuervorrichtung ebenso mit einer Ölfördervorrichtung zum Absaugen des Öls aus dem Öltank, insbesondere einer Kolbenpumpe, verbunden sein. Auf ein vom Füllstandssensor des Öltanks an die Steuervorrichtung gesendetes Signal wird die Ölfördervorrichtung in Betrieb gesetzt und Öl aus dem Öltank gefördert.

Das Verfahren zur stofflichen Behandlung von Rohstoffen kann auf dem Betreiben der beschriebenen Vorrichtung zur stofflichen Behandlung von Rohstoffen basieren. Das Verfahren weist dabei folgende Schritte auf:
- Beschicken einer Reaktionseinheit mit Rohstoffen,
- Vorwärmen der Reaktionseinheit,
- Öffnen eines Heizsystems und Verbringen der Reaktionseinheit in das Heizsystem, insbesondere auf ein Bodenelement des Heizsystems,
- Verschließen des Heizsystems, sodass die Reaktionseinheit in einem abgeschlossenen Raum angeordnet ist,
- Erwärmen der Reaktionseinheit und Starten eines Verschwelungs- und Destillationsprozesses, wobei der Verschwelungs- und Destillationsprozess durch gezieltes Beheizen bei einer im Wesentlichen konstanten Reaktionstemperatur innerhalb der Reaktionseinheit erfolgt, wobei die Temperatur bestimmt wird,
- Abführen entstehender Gase aus der Reaktionseinheit in eine Destillationseinheit durch eine zwischen der Reaktionseinheit und der Destillationseinheit ausgebildete Abgasleitung sowie Bestimmen der Temperatur des durch die Abgasleitung hindurchströmenden Gases,
- Abkühlen und Kondensieren der Gase in der Destillationseinheit, wobei durch eine Zwangskühlung einer Abkühlstrecke der Destillationseinheit die Temperatur der Gase über eine von den Gasen abgeführte Wärmeleistung gesteuert wird,
- Einleiten der Destillationsprodukte in einen Öltank und Abscheiden von Öl,
- Absaugen nichtkondensierbarer Gase aus dem Öltank, wobei innerhalb der Reaktionseinheit ein Unterdruck zur Umgebung erzeugt und Sauerstoff aus der Reaktionseinheit abgeführt wird,
- Öffnen des Heizsystems und Entnehmen der Reaktionseinheit aus dem Heizsystem,
- Abkühlen der Reaktionseinheit, Entnehmen der Endprodukte aus der Reaktionseinheit und Trennen der Endprodukte sowie
- Entnehmen der Endprodukte aus dem Öltank.

Unter dem gezielten Beheizen ist zu verstehen, dass die innerhalb des Heizsystems angeordnete Reaktionseinheit während des Verschwelungs- und Destillationsprozesses derart erwärmt wird, dass die Reaktionstemperatur innerhalb der Reaktionseinheit, auch als Prozesstemperatur bezeichnet, im Wesentlichen konstant ist und lediglich innerhalb eines vorgegebenen Temperaturbandes variiert. Dabei wird die Reaktionstemperatur permanent überwacht. Der Wert der Temperatur wird an die Steuervorrichtung übertragen, welche entsprechend eines vorgegebenen Sollwertes der Temperatur das Öffnen und Schließen der Ventile von Heizkreisen des Heizsystems und damit eine Befeuerung steuert.

Beim Verschließen des Heizsystems wird vorzugsweise ein Abgasstutzen der Reaktionseinheit mit einem Abgasstutzen einer Abgasleitung des Heizsystems sowie an einem Verbindungselement die Abgasleitung des Heizsystems und eine Abgasleitung der Destillationseinheit miteinander gekoppelt, sodass eine gasdichte Verbindung von der Reaktionseinheit zur Destillationseinheit hergestellt wird. Das Heizsystem wird vorteilhaft durch Ausfahren und Einfahren von Stützelementen geöffnet und geschlossen.

Mit dem Absaugen nichtkondensierbarer Gase aus dem Öltank und damit dem Erzeugen des Unterdrucks kann der Absolutwert des Drucks innerhalb der Reaktionseinheit im Bereich von 2 mbar bis 10mbar, insbesondere von etwa 4 mbar, eingestellt werden.

Zum Abkühlen und Kondensieren der Gase in der Destillationseinheit kann gezielt Umgebungsluft über die Abkühlstrecke der Destillationseinheit geleitet werden oder die Abkühlstrecke kann von einem flüssigen Wärmeträgerfluid, insbesondere von Wasser als Kühlmittel, durchströmt werden.

Beim Vorgang des Abkühlens und Kondensierens der Gase wird in der Destillationseinheit vorteilhaft die Temperatur der Gase, beispielsweise über einen Volumenstrom der Umgebungsluft beziehungsweise eine Leistung der Lüfter oder einen Massenstrom eines Wärmeträgerfluids, auf einen Wert in einem Bereich von 95 °C bis 125 °C eingestellt. Mit dem Volumenstrom der Umgebungsluft oder dem Massenstrom des Wärmeträgerfluids werden die von der Abkühlstrecke abzuführende Wärme sichergestellt und die Abkühlstrecke gekühlt. Die Temperatur der Gase wird in der zwischen dem Heizsystem und der Destillationseinheit ausgebildeten Abgasleitung, insbesondere dem mindestens einen Temperatursensor zum Bestimmen der Temperatur von aus dem Heizsystem abgeführter Abgase, bestimmt.

Ein Vorteil des Verfahrens besteht darin, dass während des Verschwelungs- und Destillationsprozesses eine zwischen der Reaktionseinheit und der Destillationseinheit ausgebildete Abgasleitung, insbesondere auf eine Temperatur im Bereich von 120 °C bis 160 °C, erwärmt wird, speziell um ein frühzeitiges Kondensieren des Abgases vor dem Eintritt in die Destillationseinheit und folglich ein Zusetzen der Abgasleitung zu vermeiden.

Die Reaktionseinheit wird vorzugsweise bei einer Temperatur des durch die Abgasleitung hindurchströmenden Gases von etwa 60 °C aus dem Heizsystem entnommen.

Während des Verschwelungs- und Destillationsprozesses beziehungsweise beim Vorgang des Abkühlens der Reaktionseinheit wird bevorzugt ein gasförmiges Spülmedium, insbesondere Stickstoff, in die Reaktionseinheit eingeströmt.

Das Spülen erfolgt bevorzugt jeweils in zeitlichen Intervallen, insbesondere um höhermolekulare Gase aus der Reaktionseinheit abzuführen. Mit dem Spülen mittels Inertgas, wie Stickstoff, werden insbesondere während des Verschwelungs- und Destillationsprozesses unerwünschte Komponenten, wie polyaromatische Bestandteile von Polybutadien beziehungsweise Weichmachern, aus der Reaktionseinheit entfernt. Das Absaugen nichtkondensierbarer Gase und damit das Erzeugen des Unterdrucks innerhalb der Reaktionseinheit sowie das Einströmen des Spülmediums in die Reaktionseinheit erfolgen vorteilhaft zeitlich versetzt zueinander. Speziell beim Vorgang des Abkühlens der Reaktionseinheit kann das Spülmedium periodisch jeweils für eine Dauer im Bereich von zwei bis drei Minuten in die Reaktionseinheit eingeleitet werden.

Nach dem Abkühlen der Reaktionseinheit wird die Reaktionseinheit zum Entnehmen der Endprodukte vorzugsweise bei einer Temperatur im Inneren der Reaktionseinheit im Bereich von 20 °C bis 60 °C, insbesondere im Bereich von 30 °C bis 60 °C, geöffnet. Beim Vorgang des Entnehmens der Endprodukte aus der Reaktionseinheit wird die Reaktionseinheit vorteilhaft mit dem gasförmigen Spülmedium, speziell Stickstoff, beaufschlagt.

Beim Vorgang des Entnehmens der Endprodukte aus der Reaktionseinheit kann Kohlenstoff als Endprodukt abgesaugt werden.

Nach einer Weiterbildung des Verfahrens werden abgesaugte nichtkondensierbare Gase zum Verbrennen innerhalb des Heizsystems und damit zum Beheizen der Reaktionseinheit dem Heizsystem und/oder zum Erzeugen von thermischer Energie und Elektroenergie einem Blockheizkraftwerk zugeführt.

Das Verfahren wird bevorzugt mit mindestens vier Reaktionseinheiten gleichzeitig und folgenden Schritten modular betrieben:
- Beschicken einer ersten Reaktionseinheit, während eine zweite Reaktionseinheit, welche bereits beschickt ist, vorgewärmt wird,
- Zuführen einer dritten, beschickten und vorgewärmten Reaktionseinheit dem Heizsystem und Erwärmen der Reaktionseinheit zum Durchführen des Verschwelungs- und Destillationsprozesses sowie
- Abkühlen und Entleeren einer vierten Reaktionseinheit, in welcher der Verschwelungs- und Destillationsprozess beendet ist.

Die Reaktionseinheit kann mit Rohstoffen einer Masse im Bereich von 2,5 t bis 3 t beschickt werden und verbleibt vorteilhaft für eine Dauer in einem Bereich von etwa 2,5 h bis 3,5 h im Heizsystem. Die Reaktionstemperatur innerhalb der Reaktionseinheit beträgt bevorzugt zwischen 350 °C und 800 °C, insbesondere 550 °C.

Der Energieverbrauch für einen Prozessdurchlauf mit einer insbesondere mit Altreifen bestückten Reaktionseinheit beläuft sich dabei auf 60 kWh bis 80 kWh. Bei einer Anzahl von zwölf Reaktionseinheiten und jeweils neun Durchläufen pro Tag ergibt sich ein Tagesenergiebedarf von 6.480 kWh bis 8.640 kWh. Bei durchschnittlich 223 Produktionstagen im Jahr beträgt der Jahresenergiebedarf folglich 1,445 MWh bis 1,927 MWh. Demgegenüber belaufen sich die Werte der erzeugten Energien für Strom beziehungsweise Wärme jeweils auf etwa 10,5 MWh pro Jahr.

Das Verfahren basiert auf einem Verschwelungs-Destillations-Prozess, sodass es sich bei der Vorrichtung zur stofflichen Behandlung von Rohstoffen um ein Verschwelungs-Destillations-Industriemodul, auch als VDI-Modul bezeichnet, handelt.

Zur effektiven Durchführung des Verfahrens wurde die Vorrichtung auf die Ausbildung mit Modulen gestützt, um damit den Durchsatz zu optimieren beziehungsweise zu maximieren sowie an den aktuellen Bedarf anpassen zu können.

Weitere Vorteile der Vorrichtung und dem Verfahren gegenüber dem Stand der Technik lassen sich folgendermaßen zusammenfassen:
- keine Vorsortierung der Rohstoffe,
- Behandlung von Ausgangsprodukten, insbesondere von
   - Altgummiprodukten, wie Altreifen, gummierten Kettengliedern, stahlseilbewehrten Gummigurten und Förderbändern, wobei die Produkte, um deren Struktur zu erhalten, in ihrer im Wesentlichen Ausgangsform behandelbar sind, das heißt beispielsweise weder zerkleinert noch geschreddert, und damit unzerkleinert, oder verdichtet werden,
   - organischen und nachwachsenden Rohstoffen, zum Beispiel Holz in allen Formen, insbesondere Buchen und Eichen, Bambus sowie Schalen und Früchten, wie Kokosnussschalen und Orangenschalen,
   - tierischen Abfällen, wie Knochen und Kadavern,
   - verseuchten Kohlenstoffen,
   - verseuchten Böden oder anderen Materialien, zum Beispiel nach Ölhaverien,
   - im Wesentlichen unzerkleinerten beziehungsweise unzerlegten und damit Komplett-Altfahrzeugen sowie
   - Karbonverbundmaterialien, insbesondere mit Karbonfasern, speziell aus der Automobilindustrie,
- ökologische, ökonomische sowie kohlenstoffdioxidfreie und damit nachhaltige Technologie mit sehr geringem Energieverbrauch.

Die verschiedenen Verfahrensparameter, wie die Temperaturen und die Dauer des Prozesses sowie das Spülen mit gasförmigem Spülmedium, und damit verbunden die Leistungen einzelner Komponenten, wie des Heizsystems, der Fördervorrichtungen der Vorrichtung zur Zwangskühlung der Destillationseinheit, wie den Lüftern oder der mindestens einen Pumpe, und der Absaugvorrichtung, sind von den zu behandelnden Rohstoffen innerhalb der Reaktionseinheit abhängig. So lassen sich die Verfahren beziehungsweise Vorrichtungen mit den entsprechenden in der Steuervorrichtung hinterlegten Steuerprogrammen wie folgt abgrenzen:
a) Vorrichtung und Verfahren zur stofflichen Behandlung von Reifen,
b) Vorrichtung und Verfahren zur stofflichen Behandlung von gummierten Kettengliedern,
c) Vorrichtung und Verfahren zur stofflichen Behandlung von Förderbändern,
d) Vorrichtung und Verfahren zur stofflichen Behandlung von Komplettfahrzeugen oder zerkleinerten Fahrzeugen der Automobilindustrie,
e) Vorrichtung und Verfahren zur stofflichen Behandlung von nachwachsenden Rohstoffen, wie Holz und Bambus, sowie Bioabfällen, wie Kokosnussschalen und Orangenschalen,
f) Vorrichtung und Verfahren zur stofflichen Behandlung von tierischen Abfällen,
g) Vorrichtung und Verfahren zur stofflichen Behandlung von Bitumen beziehungsweise Asphalt,
h) Vorrichtung und Verfahren zur stofflichen Behandlung von Energiespeichern, insbesondere von Batterien, speziell aus der Automobilindustrie,
i) Vorrichtung und Verfahren zur stofflichen Behandlung von Elektronikkomponenten, wie Computer, Handy, Laptop und Smartphones, sowie
j) Vorrichtung und Verfahren zur stofflichen Behandlung von verseuchtem Kohlenstoff und mit von Schadstoffen verunreinigten Böden zum Reaktivieren des Kohlenstoffs.

Je nach zu behandelnden Rohstoffen sind innerhalb der Reaktionseinheit die Rohstoffe vorteilhaft in bestimmten Verhältnissen zueinander zu mischen, beispielsweise Reifen und Batterien, um Verfahrensparameter beziehungsweise Endprodukte zu beeinflussen.

Aus der nachfolgenden Tabelle sind die rückgewonnenen Rohstoffe in mg/kg aufgeführt. Dabei sind in der dritten und vierten Spalte die Rohstoffe gemäß einer Vorrichtung und einem Verfahren nach h) und in der fünften Spalte die Rohstoffe gemäß einer Vorrichtung und einem Verfahren nach i) aufgelistet, während in der sechsten und der siebenten Spalte die Rohstoffe gemäß einer Vorrichtung und einem Verfahren nach d) sowie in der achten Spalte die Rohstoffe gemäß einer Vorrichtung und einem Verfahren nach a) aufgelistet sind.

| | **Rohstoff / mg/kg** | **E-Block 36 kg^{1,2,3}** | **E-Block 500 kg^{1,2,3}** | **E-Schrott 515 kg^{1,2,3}** | **Smart grob^{1,2,3}** | **Smart fein^{1,2,3}** | **Altreifen^{1,2,3}** |
|---|---|---|---|---|---|---|---|
| Ag | Silber | <1 | <5 | **600** | <1 | <1 | <1 |
| Al | Aluminium | **106.000** | **120.000** | **73.000** | **8.300** | **7.500** | **2.500** |
| As | Arsen | <1 | <5 | 140 | <2 | <2 | <1 |
| Ba | Barium | 19 | 9 | 2.000 | **54.000** | 40.000 | 18 |
| Be | Beryllium | <1 | 10 | 91 | <1 | <1 | - |
| Bi | Bismut | <4 | <16 | 510 | 16 | 42 | <1 |
| Ca | Calcium | 1.400 | 1.600 | **11.000** | **45.000** | **40.000** | **6.900** |
| Cd | Cadmium | <1 | <2 | **51** | <1 | <1 | 1 |
| Co | Cobalt | **54.000** | **60.000** | **36.000** | 83 | 110 | 108 |
| Cr | Chrom | 74 | 69 | **15.000** | 190 | 230 | <1 |
| Cu | Kupfer | **103.000** | **110.000** | **102.000** | 560 | 570 | 33 |
| Fe | Eisen | **28.000** | **17.000** | **122.000** | **6.500** | **6.300** | 364 |
| Ga | Gallium | <21 | <28 | **240** | <2 | <2 | <1 |
| Ge | Germanium | <6 | **260** | **350** | <1 | <1 | <1 |
| Hf | Hafnium | <2 | <9 | 76 | <1 | <1 | - |
| In | Indium | 90 | <106 | 150 | <5 | <5 | - |
| K | Kalium | <38 | <86 | 350 | 1.300 | 1.200 | 1.210 |
| Li | Lithium | **30.000** | **33.000** | **7.800** | 7 | 10 | 10 |
| Mg | Magnesium | 710 | **990** | **5.600** | **35.000** | **34.000** | 850 |
| Mn | Mangan | **46.000** | **50.000** | **12.000** | 140 | 110 | 2 |
| Mo | Molybdän | <1 | <4 | 190 | 7 | 3 | 4 |
| Na | Natrium | 180 | <14 | 1.900 | 1.500 | 2.600 | 1.300 |
| Nb | Niob | <3 | <11 | 230 | <2 | <2 | - |
| Ni | Nickel | **137.000** | **150.000** | **14.000** | 190 | 180 | <1 |
| P | Phosphor | 5.300 | 6.900 | 2.800 | 500 | 800 | 364 |
| Pb | Blei | 16 | <10 | 310 | 62 | 150 | 28 |
| Re | Rhenium | <1 | <6 | **130** | <1 | <1 | - |
| S | Schwefel | - | - | **2.600** | **24.000** | **22.000** | **25.500** |
| Si | Silicium | - | - | **27.000** | **67.000** | **63.000** | **43.500** |
| Sb | Antimon | <2 | <9 | 150 | 170 | 160 | - |
| Se | Selen | <9 | <46 | 89 | <1 | <1 | - |
| Sn | Zinn | **110** | **160** | **5.300** | 110 | 120 | 2 |
| Sr | Strontium | 8 | 31 | **2.500** | 790 | 650 | 6 |
| Ta | Tantal | <5 | <9 | **1.400** | <3 | <3 | - |
| Ti | Titan | **240** | **280** | **1.500** | **3.200** | **3.100** | 208 |
| Tl | Thallium | - | <15 | 180 | <14 | <14 | <1 |
| V | Vanadium | <5 | <3 | 190 | 14 | 15 | 6 |
| Zn | Zink | **250** | **300** | **4.700** | **14.000** | **17.000** | **39.500** |
| Zr | Zirconium | 36 | 55 | **680** | 16 | 12 | - |
| | | | | | | | |
| C | Kohlenstoff | 29,1 % | 29,5 % | 10,2% | 49,7 % | 49,3 % | |

| | | | | | | | |
|---|---|---|---|---|---|---|---|
| ¹Gemahlen auf Partikelgrösse <0,1 mm - Ergebnisse head space GC-MS screening sowie Thermogravimetrie-Ergebnisse ²TGA Graph, Ergebnisse GC-MS screening Spurenelemente mittels ICP OES nach HN03/HF Säureaufschluss - SOP 671 (679) | | | | | | | |

Beim Verfahren nach h), dessen Rohstoffe in der vierten Spalte der Tabelle aufgelistet sind, wurden als Ausgangsmaterial Batterieblocks, auch als Energie-Blocks bezeichnet, aus der Automobilindustrie mit einer Masse von 500 kg sowie Altreifen mit einer Masse von etwa 500 kg verwendet. Vor dem Prozess wurden von den Batterieblocks die Stahlummantelungen inklusive Schrauben mit einer Masse von etwa 60 kg entfernt und die verbliebenen 440 kg Ausgangsmaterial auf ein separates Sieb geschichtet, um ein Vermischen der Batterieblocks mit den Altreifen innerhalb der Reaktionseinheit zu vermeiden. Die nach dem Ende des abgelaufenen Prozesses aus der Reaktionseinheit entnommenen Rückstände der verarbeiteten Batterieblocks wiesen eine Masse von 220,9 kg auf und wurden zur weiterführenden Analyse auf eine einheitliche Größe im Bereich von 0,2 mm bis 0,5 mm geschreddert. Aus den in der Tabelle aufgeführten Analysedaten ist zu entnehmen, dass sämtliche anorganischen oder metallischen Bestandteile der Batterieblocks in einer Wiederfindungsrate von über 98,5 % nachgewiesen werden. Die Metalle und anorganischen Bestandteile, wie Kobalt, Nickel, Magnesium, Kupfer, Niob und Lithium, sind durch bewährte Metall-Raffinierung rückzugewinnen.

Beim Verfahren nach i), dessen Rohstoffe in der fünften Spalte der Tabelle aufgelistet sind, wurden als Ausgangsmaterial Elektroschrott, wie Fernseher, Bohrmaschinen und Kabel, mit einer Masse von 500 kg, Elektronikschrott, wie Computer in Form von Laptops und Mobiltelefonen, mit einer Masse von 15 kg sowie Altreifen mit einer Masse von etwa 500 kg verwendet. Die Computer und Mobiltelefone wurden in einer Metallbox gesondert in die Reaktionseinheit verbracht, um ein Vermischen mit den anderen Ausgangsmaterialien zu vermeiden. Die nach dem Ende des abgelaufenen Prozesses aus der Metallbox entnommenen Rückstände der verarbeiteten Computer und Mobiltelefone wiesen eine feste Masse von 7,7 kg auf und wurden zur weiterführenden Analyse auf eine einheitliche Größe im Bereich von 0,1 mm geschreddert. Die optische Emissionsanalyse ergab eine hohe Wiederfindungsrate der Metalle, wie Kobalt, Chrom, Lithium, Nickel, Cadmium, Tantal, Gallium, Germanium, Mangan, Rhenium, Strontium und Zirkonium, welche durch bewährte Metall-Raffinierung rückzugewinnen sind. Es ist eine Wiederfindungsrate beziehungsweise Verwertungsrate von 98 % festzustellen.

Beim Verfahren nach d), dessen Rohstoffe in der sechsten Spalte der Tabelle aufgelistet sind, wurde als Ausgangsmaterial ein Komplettfahrzeug der Marke Smart mit einer Masse von 750 kg für den Prozess verwendet. Vor dem Prozess wurden lediglich die Flüssigkeiten, wie die Kühlflüssigkeit und die Bremsflüssigkeit, das Motoröl und das Benzin, sowie die Batterie entfernt. Die nach dem Ende des abgelaufenen Prozesses aus der Reaktionseinheit entnommenen festen Rückstände des verarbeiteten Komplettfahrzeugs wiesen eine Masse von 450 kg auf. Dabei war die Masse aus 30 % Kohlenstoff und 70 % Metallen, wie Stahl, Federstahl und Edelmetalle, zusammengesetzt. Zudem wurde etwa 250 kg bis 270 kg leichtes Öl zurückgewonnen. Der Anteil an Restgas betrug etwa 6 % bis 8 %. Damit ergibt sich eine Wiederfindungsrate beziehungsweise Verwertungsrate von 95 %.

Bei Proben aus Raps - ungemahlen oder gemahlen - werden mit dem Verfahren nach DIN/EN 12879 Kohlenstoffgehalte zwischen 98,8 % bis 99,8 % ermittelt, während jeweils mit dem gleichen Verfahren bei Proben aus Rapspellets zu Ruß Kohlenstoffgehalte im Bereich von 79,7 % bis 81,0 %, bei Proben aus Plastikflaschen ein Kohlenstoffgehalt von 99,1 %, bei Proben aus Eichenholz ein Kohlenstoffgehalt von 98,5 %, bei Proben aus Industrieabfällen ein Kohlenstoffgehalt von 99,4 % und bei Proben aus Gummiabfällen ein Kohlenstoffgehalt von 99,4 % bestimmt werden. Bei Proben aus Rapspellets zu Öl wird ein Kohlenstoffgehalt von 99,5 % ermittelt.

Weitere wesentliche Vorteile sind, dass die bisher nur mit energetisch hohem Aufwand trennbaren Stahl-Gummi-Verbunde ohne wesentliche Inanspruchnahme von Fremdenergie trennbar sind. Die entstehenden Produkte sind im Sinne einer effizienten Kreislaufwirtschaft wieder einer hochwertigen Nutzung zuführbar, welche dazu beiträgt, Ressourcen zu schonen. Zudem erschließen sich neuartige Verwendungen der durch das Verfahren gewonnenen Materialien, wobei die entstehenden Produkte auf unterschiedlichen prozentualen Verteilungen beruhen, welche wiederum auf den unterschiedlich eingesetzten Rohstoffen basieren. Zu den entstehenden Produkten zählen:
- leichtes Öl, beispielsweise mit einer Dichte von etwa 927 kg/m³ bei 15 °C, einer Viskosität von 4,74 mm²/s und einem Flammpunkt unter 21 °C,
- Gas,
- Metalle, überwiegend Stahl beziehungsweise Eisen und Titan, sowie
- amorpher Kohlenstoff beziehungsweise Kohlenstoffagglomerate.

Der mit dem Verfahren zur stofflichen Behandlung von Rohstoffen hergestellte Kohlenstoff weist konzeptionsgemäß eine Struktur einer dreidimensionalen Anordnung von Kohlenstoffnanoteilchen und je nach Ausgangsrohstoffen vorteilhaft einen Reinheitsgrad im Bereich von 95 % bis 99,9 % auf. Der mit der Struktur aus dreidimensional angeordneten Nanoteilchen ausgebildete Kohlenstoff wird mittels der Vorrichtung beziehungsweise dem Verfahren zur stofflichen Behandlung von Rohstoffen industriell erzeugt und weist damit einen wesentlichen ökonomischen Vorteil gegenüber aus dem Stand der Technik bekannten im Labor gewonnenen oder hergestellten Kohlenstoffen auf. Die Reinheit des Kohlenstoffs wird maßgeblich durch das Spülen mit gasförmigem Spülmedium, insbesondere während des Verschwelungs- und Destillationsprozesses beziehungsweise des Abkühlens der Reaktionseinheit, beeinflusst.

Der beim Verfahren zur stofflichen Behandlung von Rohstoffen hergestellte Kohlenstoff weist bevorzugt eine BET-Oberfläche im Bereich von 2.500 m²/g BET bis 8.000 m²/g BET, insbesondere im Bereich von 4.200 m²/g BET bis 4.500 m²/g BET, und damit ein sehr hohes Adsorptionsvermögen ohne Abgabe von Substanzen an die Umwelt auf. Damit wird die Umwelt, zum Beispiel durch Auswaschungen, nicht belastet.

Der mit dem Verfahren hergestellte Kohlenstoff weist vorzugsweise eine Dichte von etwa 66 kg/m³ auf und kann vorteilhaft mit einer größeren Zugfestigkeit als legierter Stahl ausgebildet sein. Der derart gewonnene Kohlenstoff kann über eine elektrische Leitfähigkeit im Bereich von 4,5·10⁷ Ωm bis 5,8·10⁷ Ωm verfügen.

Der beim erfindungsgemäßen Verfahren zum Betreiben der Vorrichtung zur stofflichen Behandlung von Rohstoffen entstehende Kohlenstoff ist in konzentriert oder verdünnten kalten Säuren, wie Schwefelsäure, Salpetersäure, Salzsäure, nicht löslich und wird durch Laugen nicht angegriffen. Salpetersäure wird spontan in Wasser und nitrose Gase zersetzt, was auf eine katalytische Wirkung hinweisen kann. Weder polare organische Lösungsmittel noch unpolare Lösungsmittel vermögen den Kohlenstoff zu lösen.

Der mit dem Verfahren hergestellte amorphe Kohlenstoff kann beispielsweise in der Lebensmittelindustrie und in der Medizin, in Entkalkungssystemen, zur Diamantenherstellung, als Füllstoff für Kautschuk bei der Gummiherstellung und Reifenherstellung, im Flugzeugbau, in der Bauindustrie und zum Herstellen von Speichersystemen für elektrische Energie, wie Akkumulatoren oder Batterien beziehungsweise Kondensatoren, Verwendung finden.

Da der mit dem Verfahren hergestellte und gegebenenfalls gereinigte amorphe Kohlenstoff keine akute, unmittelbare Zelltoxizität aufweist, ist die Verwendung des Kohlenstoffs in der Medizin möglich. Bei einer Inkubation von Kardiomyozyten mit dem Kohlenstoff ergibt sich, dass die Kardiomyozyten nicht negativ beeinflusst werden und deren Kontraktilität erhalten bleibt.

Der Kohlenstoff kann zur Hämoperfusion/-adsorption und damit zum Reinigen von Blut, auch als Dialyse bezeichnet, eingesetzt werden. Dabei steht das Entfernen beziehungsweise Verringern von schädlichen Plasmabestandteilen, welche entweder in Folge von pathologischen Veränderungen, durch übermäßige Aufnahme in den Organismus oder durch mangelnde Elimination bei einem Ausfall der Nierenfunktion oder der Leberfunktion anfallen.

Das zur reinigende Blut wird durch eine mit dem Kohlenstoff befüllte Kartusche geleitet. Die Kartusche weist eine Form auf, welche eine größtmögliche Oberfläche für den Kontakt des Kohlenstoffs mit dem Blut ermöglicht. Der Auslass der Kartusche ist mit einer doppelt gesicherten Filtermembran versehen, sodass keine Kohlenstoffpartikel den Auslass passieren können und der Kohlenstoff gesichert in der Kartusche verbleibt. Die Filtermembran ist derart konfiguriert, die zellulären Bestandteile des Blutes, wie rote Blutkörperchen mit einer Große von etwa 7,5 µm, Blutplättchen mit einer Größe im Bereich von 1 µm bis 4 µm, weiße Blutkörperchen mit einer Größe von 7 µm bis 20 µm, beziehungsweise essentielle Plasmabestandteile, wie Albumin 40.000 Dalton bis 50.000 Dalton, hindurchzulassen. Nach einer alternativen Ausführungsform ist der Kohlenstoff durch ein Bindungsverfahren auf einer Oberfläche sicher fixiert. Mit dem Kohlenstoff beschichtete Elemente sind innerhalb der Kartusche angeordnet, welche vom Blut durchströmt wird.

Der Kohlenstoff kann ebenso zur topischen Anwendung auf der Haut, insbesondere zur Wundheilung, speziell zur Behandlung von Wunden und Wundoberflächen, verwendet werden. Dabei ist der Kohlenstoff auf synthetischen Oberflächen, wie Pflastermaterialien, fixierbar.

Zudem ist der Kohlenstoff zur oralen Aufnahme als Antidot, als Trägermolekül beispielsweise für Antibiotika, zum Beschichten für eine verbesserte Gleitfähigkeit und für Implantatbeschichtungen geeignet.

Der Kohlenstoff kann als Applikation bei Vergiftungen, insbesondere zur primären und sekundären Giftentfernung bei Vergiftungen auch mit Säuren oder Laugen, Zyaniden, Alkoholen, wie Ethanol, Methanol, und Glykolen, organischen Lösungsmitteln, wie Aceton und Dimethylsulfoxid, anorganischen Salzen oder Metallen, wie Lithium, Eisen oder anderen Schwermetallen, wie Blei oder Quecksilber, dienen. Der Kohlenstoff kann in Form von Tabletten oder Pulver beziehungsweise Granulat verabreicht werden. Dabei kann der Kohlenstoff in einer Flüssigkeit aufgeschlämmt eingenommen werden. Die Adsorptionskapazität des Kohlenstoffs für Fette und andere Substanzen könnte beispielsweise auch zum Entlasten der Leber von einem Teil der Entgiftungsfunktion und der Bauspeicheldrüse von einem Teil der sekretorischen Funktion führen.

Der mit dem Verfahren hergestellte amorphe Kohlenstoff kann, wie oben erwähnt, auch als ein Speicherelement, insbesondere als eine Komponente eines elektrischen Energiespeichers, wie einer Batterie oder eines Kondensators, oder als eine Komponente eines Datenspeichers, verwendet werden. Das Speicherelement ist dann beispielsweise in einem Kraftfahrzeug, in der Luftfahrtelektronik oder der Satellitenelektronik verwendbar.

So stellen sogenannte Superkondensatoren, englisch "Supercapacitors" oder kurz als Supercaps bezeichnet, als elektrochemische Kondensatoren eine elektrische Speichervorrichtung beziehungsweise einen elektrischen Energiespeicher als eine Verbindung aus einem Kondensator und einer chemischen Batterie dar. Bei einem Kondensator wird bekanntlich zwischen zwei Flächen eine elektromagnetische Energie gespeichert. Mit der großen BET-Oberfläche als massenbezogene spezifische Oberfläche des Kohlenstoffs mit der Struktur der dreidimensionalen Anordnung von Kohlenstoffnanoteilchen wird eine hohe Energiedichte auf minimalem Raum ermöglicht.

Mit der chemischen Erweiterung zum Superkondensator wird einerseits die Energie-Kapazität erhöht und andererseits eine höhere Stabilität erreicht. Die Stabilität bezieht sich dabei auf ein automatisches Selbstentladen des Energiespeichers, welches bei einer hohen Stabilität verhindert wird.

Eine Speichervorrichtung ist erfindungsgemäß als elektrischer Energiespeicher in Form eines Doppelschicht-Kondensators mit einem symmetrischen Aufbau mit von außen nach innen jeweils einem Gehäuse und einem Kollektor mit einer als Kohlenstoffschicht ausgebildeten Elektrode und einem Separator mit einem Elektrolyten ausgebildet. Dabei ist die Kohlenstoffschicht aus einem mit dem Verfahren zur stofflichen Behandlung von Rohstoffen hergestellten Kohlenstoff mit der Struktur der dreidimensionalen Anordnung von Kohlenstoffnanoteilchen ausgebildet.

Der Kohlenstoff kann als Filter zur Wasseraufbereitung oder zur Gasreinigung in Abluftanlagen eingesetzt werden. Mit Hilfe der Filter lässt sich vorteilhaft Salzwasser in Süßwasser umwandeln und Öl, Benzin oder Säure beziehungsweise Iod aus Wasser ausfiltern. Zum Beispiel wird der Kohlenstoff zum Entsalzen von Salzwasser mit Zellulose vermischt - der derartige Filter weist dann die Form einer Tüte, speziell einer Filtertüte, auf.

Eine Untersuchung eines Gemischs aus zwei Liter Leitungswasser und 500 ml Betadine, das heißt Iod, versetzt mit 500 mg des Kohlenstoffs, zeigt einen Wert kleiner 0,1 mg/Liter I₂ (Iod). Das gleiche Ergebnis wird auch bei jeweils um drei Monate zeitlich versetzten Nachuntersuchungen des Gemischs erzielt. Die auf einer fotometrischen Analyse von Iod basierenden Untersuchungen zeigen, dass der in das Gemisch eingebrachte Kohlenstoff das Iod bindet und dort nicht wieder freisetzt.

Das Einbringen des Kohlenstoffs in Wasser verbessert zudem die Wasserqualität bezüglich des Sauerstoffgehaltes - es fördert den Sauerstoffaustausch, zum Beispiel beim Einsatz in einem Aquarium. Ein weiterer Vorteil liegt darin, dass beispielsweise Koli-Bakterien erst bei erhöhten Temperaturen des Wassers von etwa 36 °C bis 38 °C und darüber aktiv werden. Bei Temperaturen unterhalb des angegebenen Bereiches werden dagegen keine Koli-Bakterien gebildet.

So weist beispielsweise ein Wasserfiltersystem ein Filterelement, bestehend aus 40 kg des Kohlenstoffs, welcher in vier Einheiten unterteilt ist, auf. Das Wasserfiltersystem dient der Reinigung eines Volumens von etwa 4 Mio. Liter Wasser, beispielsweise eines Wasserbeckens eines Bades oder eines Sees.

Der mit dem Verfahren hergestellte amorphe Kohlenstoff kann als Filterelement ebenso zur Reinigung von Luft in Klimaanlagen, beispielsweise von Immobilien, wie Krankenhäusern, Wohnhäusern, Fabriken, Hallen oder Ähnlichen, und von mobilen Einheiten, wie Fahrzeugen oder Flugzeugen, eingesetzt werden. Zudem ist die Verwendung des Kohlenstoffs als Luftfilterelement in Atemschutzmasken oder einer Abgasvorrichtung, beispielsweise eines Kraftfahrzeugs, möglich.

Aufgrund seiner Eigenschaften ist der Kohlenstoff zur Ölbekämpfung geeignet. Der Kohlenstoff schwimmt auf der Wasseroberfläche auf und bindet auf dem Wasser befindliches Öl, wie es zum Beispiel bei Seeunfällen auftreten kann. Eine Wasserkontamination kann bekämpft beziehungsweise verhindert werden. Dabei nimmt Filtermaterial aus 1 kg Kohlenstoff etwa 3,33 Liter Öl auf. Wenn folglich 1 Liter Öl etwa 1 Mio. Liter Wasser kontaminiert, können mit einem Filtermaterial aus 3 kg Kohlenstoff 10 Liter Öl aufgenommen werden und derart 10 Mio. Liter Wasser gereinigt werden.

Gleichfalls ist der Kohlenstoff aber auch zur Säuberung von mineralölverseuchten Böden, das heißt bei Bodenkontaminationen, oder bei anderen Ölschadensfällen beziehungsweise kontaminierten Stoffen einsetzbar. Der Kohlenstoff weist ein sehr gutes Elutionsverhalten auf und verhindert folglich ein Herauslösen bereits adsorbierter Stoffe, insbesondere in Wasser. Damit wird eine Belastung von Boden und Grundwasser durch mögliche Auswaschungen von Schadstoffen aus dem Kohlenstoff vermieden.

Ebenso wird der Kohlenstoff vorteilhaft zur Brandbekämpfung auf dem Land und im Wasser, insbesondere für die Bekämpfung von brennendem Öl, eingesetzt. Der Kohlenstoff ist somit einerseits als Löschmittel einsetzbar, wobei dem Feuer durch Abdecken mit einer entsprechenden Menge an Kohlenstoff Sauerstoff entzogen wird, sodass die Flamme erstickt. Andererseits wird mit dem Kohlenstoff das Öl gleichzeitig gebunden.

Der mit dem Verfahren hergestellte amorphe Kohlenstoff kann ebenso zum Löschen von Waldbränden eingesetzt werden, behindert die Verbreitung schädlicher Pilze aus der Holzasche und filtert freigesetzte Giftstoffe, wie Blei, Quecksilber, Schwefel und Dioxin.

Eine weitere Anwendung des Kohlenstoffs dient dem Brandschutz sowie der Wärmeisolierung bis mindestens 3.500 °C. Unter Wärmeisolierung ist dabei auch die Isolierung bei sehr geringen Temperaturen, das heißt eine Kälteisolierung zu verstehen. Untersuchungen einerseits mit Plasmastrahlen als thermische Anregung und andererseits mit flüssigem Stickstoff zeigen, dass mit dem Kohlenstoff beschichtete Basismaterialien Temperaturen einerseits im Bereich von 2.000 °C bis 12.000 °C sowie andererseits bis -196 °C nicht nur standhalten, sondern eine thermische Isolation darstellen.

Eine Beschichtung von Glas mit Kohlenstoff führt beispielsweise zu einer Erhöhung des Feuerwiderstandes verbunden mit einer thermischen Isolationswirkung.

Eine Mischung aus Zement und Kohlenstoff im Volumen-Verhältnis im Bereich von 2:1 bis 5:1 weist sehr gute Eigenschaften bezüglich des Wärmewiderstandes beziehungsweise der Wärmeisolation auf. So ist beispielsweise eine leichtgewichtige Platte aus Kohlenstoff-Zement mit einem Mischungsverhältnis von Kohlenstoff und Zement von 3:1, einer Dicke von 10 mm bis zu Temperaturen von etwa 2.000 °C wärmeresistent. Dabei wird die Platte auf einer ersten Seite mit einer Propangasflamme auf eine Temperatur zwischen 1.500 °C bis 2.500 °C erhitzt, ohne dass auf der der ersten Seite gegenüberliegenden zweite Seite eine fühlbare Wärmeentwicklung zu verzeichnen ist, was unter thermischer Isolation zu verstehen ist.

Je nach Trägermaterial kann eine Mischung des Materials mit dem Kohlenstoff bei einem Anteil von 20 % Kohlenstoff bis zu einer Temperatur von mindestens etwa 2.500 °C wärmeresistent sein.

Neben Zement beziehungsweise Beton können beispielsweise auch Gips und Ton, Farben und Lacke sowie Sägespäne als Mischungskomponente mit dem Kohlenstoff dienen.

Zudem kann der strahlenabweisende Kohlenstoff in Anlagen und Vorrichtungen mit notwendigem Strahlenschutz eingesetzt werden. Die vorteilhaften Eigenschaften, wie die Strahlungsresistenz und die Feuerresistenz, führen zum Beispiel zum Einsatz beim Bau von Umhausungen für Nuklearreaktoren.

Ein weiteres Anwendungsgebiet für den Kohlenstoff als sehr guter Wasserspeicher und Nährstoffspeicher liegt in der Bereitstellung von Wasserhaltungsschichten. Dies führt bei bewirtschafteten Flächen, beispielsweise zum Herstellen von Nahrungsmitteln, zu einer Wasserersparnis von 60 % bis 80 %, insbesondere bei einer internen Bewässerung.

Durch den Einsatz des Kohlenstoffs, zum Beispiel unterhalb von Sandschichten, können Wasser und Pflanzennährstoffe gespeichert und karge, minderwertige Böden als Standort für Gemüse und andere landwirtschaftliche Produkte genutzt werden. Diese Anwendung ist somit für die Urbarmachung von Wüstengebieten, im Gartenbau und in der Landwirtschaft von großem Vorteil. Der Kohlenstoff gibt bekanntlich dabei zudem keine Substanzen an das Wasser ab, sodass keine Belastung des Bodens und des Grundwassers durch eine Auswaschung von Schadstoffen erfolgt.

Der mit dem Verfahren hergestellte amorphe Kohlenstoff kann folglich im Bereich der Pflanzenproduktion der Landwirtschaft und der Umweltpflege des Landschaftsbaus zur Förderung des Wasserhaltevermögens des Bodens durch Anreichern der obersten Bodenschichten mit dem Kohlenstoff verwendet werden.

Durch das Verbessern der Wasserspeicherung wird die optimale Versorgung der Pflanzen mit Wasser und damit eine Verfrühung sowie Steigerung der Qualität und Quantität des Ertrages bewirkt. Da das Versickern des Wassers in tiefere Schichten verhindert und im Bereich der Wurzeln der Pflanzen gehalten wird, können gleichzeitig länger andauernde Trockenperioden überbrückt werden. Der beschriebene Effekt, welcher auch zur Begrünung von Wüsten, Steppen und Savannen dient, kann insbesondere durch ein großflächiges Einbringen einer oder mehrerer Schichten des Kohlenstoffs in leichten Böden in einer Tiefe von etwa 20 cm bis 30 cm erzielt werden.

Die den pH-Wert beeinflussende Kohlenstoffschicht verbessert die Durchlüftung des Bodens durch Freisetzen von gebundenem Sauerstoff und Stickstoff, fördert das Anreichern von Mikroorganismen und optimiert die Lebensbedingungen der Mikroorganismen. Zudem werden mit der Kohlenstoffschicht die Versorgung der Böden und Pflanzen mit Mineralstoffen, Spurenelementen und Mikronährstoffen verbessert sowie die Beschleunigung des Reifeprozesses und des Geschmackes der Früchte ohne toxische Beeinträchtigungen gefördert. Die Kohlenstoffschicht unterstützt die Regelung der Temperaturverhältnisse im Boden und verbessert die Pufferungseigenschaften des Bodens.

Des Weiteren kann der Kohlenstoff als nachhaltiger natürlicher Halmstabilisator in der Getreideproduktion eingesetzt werden. Der Kohlenstoff übernimmt die Funktion von Leguminosen, ohne diese in der Fruchtfolge real anbauen zu müssen.

Das rückgewonnene leichte Öl dient beispielsweise der Verwendung in der chemischen Industrie, insbesondere als Rohstoff für Basischemikalien, und in der Pharmaindustrie, zum Erzeugen von thermischer Energie und Elektroenergie zum Beispiel mittels eines BHKW, während das Gas zum Erzeugen von thermischer Energie und Elektroenergie zum Beispiel mittels Gasturbine und Generator oder zur Rückführung und Nutzung im Prozess genutzt werden kann. Die rückgewonnenen Metalle, wie Stahl, können der Stahlindustrie - durch sehr geringe Prozesstemperaturen können Metalle in ihren physikalischen und chemischen Eigenschaften erhalten bleiben - zurückgeführt werden.

Weitere Einzelheiten, Merkmale und Vorteile der Erfindung ergeben sich aus der nachfolgenden Beschreibung von Ausführungsbeispielen mit Bezugnahme auf die zugehörigen Zeichnungen. Es zeigen:
- Figur 1:: Verschwelungs-Destillations-Industriemodul als Vorrichtung zur stofflichen Behandlung von Rohstoffen im geöffneten Zustand in Vorderansicht,
- Figur 2a:: Verschwelungs-Destillations-Industriemodul als Vorrichtung zur stofflichen Behandlung von Rohstoffen im geschlossenen Zustand in Seitenansicht sowie
- Figur 2b:: in Vorderansicht,
- Figur 3:: Schnittdarstellung des Heizsystems in geöffnetem Zustand,
- Figur 4:: Schnittdarstellung des Heizsystems in geschlossenem Zustand,
- Figur 5:: Bodenelement des Heizsystems,
- Figur 6:: Destillationseinheit,
- Figur 7:: Öltank sowie
- Figur 8a:: Reaktionseinheit in geschlossenem Zustand und
- Figur 8b:: Schnittdarstellung der Reaktionseinheit in geschlossenem Zustand,
- Figuren 9a bis 9n:: mikroskopische Aufnahmen von mit der Vorrichtung zur stofflichen Behandlung von Rohstoffen erzeugtem Kohlenstoff sowie
- Figuren 9p und 9q:: Ergebnisse Raman-Spektroskopie des Kohlenstoffs und
- Figur 10:: einen elektrischen Energiespeicher als Speichervorrichtung in Form eines Superkondensators in Schichtbauweise.

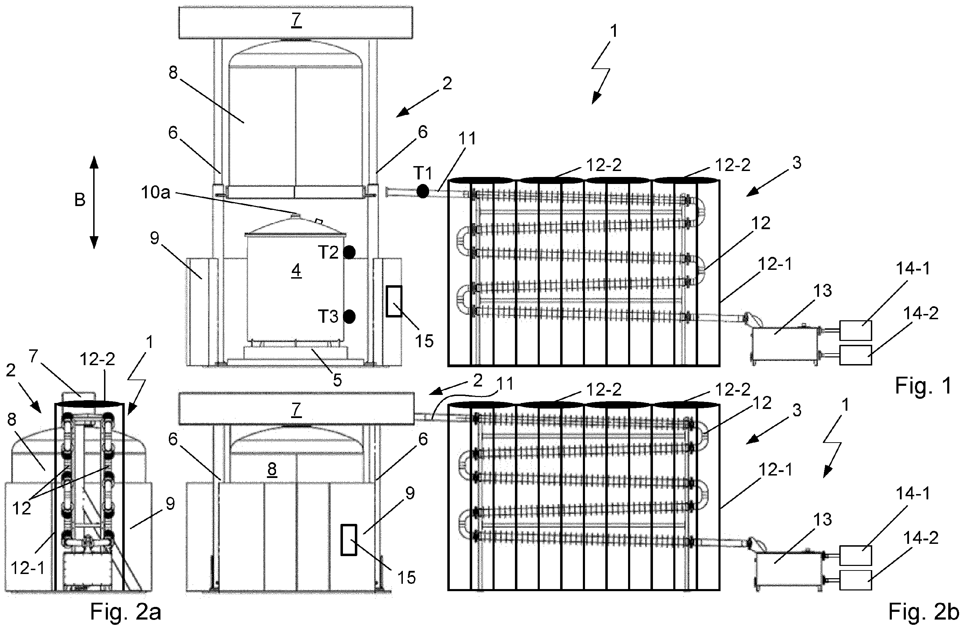

In den **Figuren 1, 2a und 2b** ist ein Verschwelungs-Destillations-Industriemodul als Vorrichtung 1 zur stofflichen Behandlung von Rohstoffen dargestellt. Figur 1 zeigt die Vorrichtung 1 im geöffneten Zustand in Vorderansicht, während aus Figur 2b die Vorrichtung 1 im geschlossenen Zustand in Vorderansicht und aus Figur 2a in Seitenansicht hervorgeht.

Die Vorrichtung 1 weist ein Heizsystem 2 sowie eine Destillationseinheit 3 auf. Die mit Rohstoffen beschickte Reaktionseinheit 4 wird in einer nicht dargestellten Vorwärmeinrichtung auf eine bestimmte Temperatur vorgewärmt und anschließend im Heizsystem 2 weiter erhitzt. Die Reaktionseinheit 4 kann dabei mit einer Mischung aus verschiedenen Rohstoffen beschickt sein, sodass keine Vorsortierung der Produkte notwendig ist. Die Reaktionseinheit 4 wird nach dem Vorwärmen in das geöffnete Heizsystem 2 verbracht und auf dem Bodenelement 5 des Heizsystems 2 positioniert.

Das Kopfelement 7 und das mit dem Kopfelement 7 fest verbundene Mantelelement 8 des Heizsystems 2 sind mittels beiderseits des Heizsystems 2 angeordneter Stützelemente 6 in der Bewegungsrichtung B beweglich gehaltert. Die Stützelemente 6 sind etwa 2,9 m zueinander beabstandet angeordnet. Das Mantelelement 8 weist einen Außendurchmesser von etwa 2,5 m auf.

In der ersten Endposition gemäß Figur 1 sind die Stützelemente 6 ausgefahren. Die Vorrichtung 1 weist dabei eine Höhe von etwa 6,70 m auf. Das Kopfelement 7 und das Mantelelement 8 geben den Raum zur Bestückung des Heizsystems 2 mit der Reaktionseinheit 4 frei. Das Heizsystem 2 ist geöffnet. Die Reaktionseinheit 4 kann in das Heizsystem 2 eingebracht oder aus dem Heizsystem 2 entnommen werden. Die Bewegung der Reaktionseinheit 4 kann dabei vorteilhaft mittels eines nicht dargestellten Schienensystems erfolgen, auf welchem die Reaktionseinheit 4 aufsteht. In der zweiten Endposition gemäß den Figuren 2a, 2b sind die Stützelemente 6 eingefahren. Die Vorrichtung 1 weist dabei eine Höhe von etwa 3,70 m auf. Das Mantelelement 8 sitzt auf dem Bodenelement 5 derart auf, dass die Reaktionseinheit 4 in einem abgeschlossenen Raum positioniert ist. Das Heizsystem 2 ist geschlossen. Die Reaktionseinheit 4 ist am Boden vom Bodenelement 5 sowie an der Seitenfläche und oben vom Mantelelement 8 umgeben.

Die Vorrichtung 1 weist im Bereich des Heizsystems 2 und der Destillationseinheit 3 Temperatursensoren T1, T2, T3 zum Ermitteln bestimmter Prozesstemperaturen auf. Dabei sind mindestens zwei Temperatursensoren T2, T3 in einem im geschlossenen Zustand des Heizsystems 2 zwischen der Reaktionseinheit 4 und dem Mantelelement 8 ausgebildeten Zwischenraum angeordnet. Die Temperatursensoren T2, T3 sind beispielsweise von der Innenseite des Mantelelements 8 etwa 1 cm in den etwa 8 cm breiten Zwischenraum hineinragend positioniert. Die Temperatursensoren T2, T3 sind in vertikaler Richtung beabstandet zueinander angeordnet, um lokale Temperaturwerte beziehungsweise eine mittlere Temperatur innerhalb des Zwischenraums zu ermitteln. Mit den über die Temperatursensoren T2, T3 ermittelten Temperaturwerten wird die Temperatur innerhalb der Reaktionseinheit 4 bestimmt.

Das Heizsystem 2 weist im unteren Bereich eine Umhausung 9 auf. Die das Bodenelement 5 sowie die Seitenflächen des Mantelelementes 8 im geschlossenen Zustand des Heizsystems 2 umschließende Umhausung 9 wird zur Bestückung des Heizsystems 2 geöffnet.

Die im Verschwelungsprozess entstehenden Gase werden durch die vorgesehene Abgasleitung 11 aus dem Heizsystem 2 abgeführt und verfahrenstechnisch abgekühlt. Die Gase werden dabei durch den am obersten Punkt der Reaktionseinheit 4 ausgebildeten Abgasstutzen 10a sowie die im Kopfelement 7 angeordnete Abgasleitung 11 zur Destillationseinheit 3 geleitet.

Anschließend durchströmen die Gase die Abkühlstrecke 12 der Destillationseinheit 3. Die Abkühlstrecke 12 ist nach den Figuren 1, 2a, 2b aus Rohren ausgebildet. Die zur Horizontalen geneigt angeordneten Rohre sind zur Vergrößerung der Wärmeübertragungsfläche und damit der Verbesserung des Wärmeüberganges mit Rippen versehen. Die Wärme wird dabei von den Gasen an die Umgebungsluft übertragen.

Zum weiteren Erhöhen der zu übertragenden Wärmeleistung von den abzukühlenden Gasen an die Umgebungsluft und speziell zum Verbessern der Steuerung der Temperatur der durch die Abkühlstrecke 12 der Destillationseinheit 3 hindurchströmenden Gase ist die Abkühlstrecke 12 mit einem Luftleitgehäuse 12-1 umgeben. An der Oberseite, insbesondere an der in vertikaler Richtung nach oben weisenden Stirnfläche des Luftleitgehäuses 12-1 sind Lüfter 12-2 angeordnet, welche die Umgebungsluft als Kühlluft gleichmäßig durch das Luftleitgehäuse 12-1 hindurch ansaugen. Alternativ können die Lüfter auch an einer Seitenfläche des Luftleitgehäuses 12-1 ausgebildet sein. Dabei wird die Umgebungsluft gezielt über die Abkühlstrecke 12 geleitet. An der zwischen dem Heizsystem 2 und der Destillationseinheit 3 ausgebildeten Abgasleitung 11 ist ein weiterer Temperatursensor T1 angeordnet, um die Temperatur der aus dem Heizsystem 2 abgeführten Abgase zu bestimmen.

Nach einer alternativen Ausgestaltung können die Gase innerhalb der Abkühlstrecke auch mit einem anderen Wärmeträgerfluid als Luft, zum Beispiel Wasser, gekühlt werden. Dabei ist die Abkühlstrecke anstelle von Rohren mit an der äußeren Mantelfläche ausgebildeten Rippen aus Koaxialrohren ausgebildet. Die Gase strömen im Inneren des innenliegenden Rohres, während das vorzugsweise flüssige Wärmeträgerfluid im Zwischenraum zwischen der Außenseite des innenliegenden Rohres und der Innenseite des außenliegenden Rohres hindurchgeleitet wird.

Die Abkühlstrecke 12 ist mit zwei parallel zueinander ausgerichteten Rohren ausgebildet. Die Gase werden vor dem Eintritt in die Abkühlstrecke 12 auf zwei Teilmassenströme aufgeteilt und nach dem Durchströmen der Abkühlstrecke 12 wieder vermischt.

Anschließend werden die Destillationsprodukte in einen Öltank 13 eingeleitet. Im Öltank 13 setzt sich das aus dem Verschwelungsprozess und der nachfolgenden Destillation gewonnene Öl, welches in seiner Konsistenz und Zusammensetzung einem leichten Öl entspricht beziehungsweise den Zwischenprodukten der Rohölverarbeitung sehr ähnlich ist, ab. Der nicht kondensierbare Anteil des Gases wird vom Öltank 13 abgeführt. Der Öltank 13 dient mit einem Fassungsvermögen von etwa 1.000 Litern auch als Ausdehnungsgefäß der Vorrichtung 1.

Am Öltank 13 ist eine Absaugvorrichtung 14-1, insbesondere eine Pumpe, speziell eine Membranpumpe, zum Absaugen der Gase über die Oberfläche des sich innerhalb des Öltanks 13 ansammelnden Öls, und eine Ölfördervorrichtung 14-2, insbesondere eine Pumpe, speziell eine Kolbenpumpe, zum Absaugen des Öls aus dem Öltank 13 angeordnet. Mit dem Absaugen der Gase wird innerhalb der Abkühlstrecke 12 der Destillationseinheit 3, der Abgasleitung 11 und speziell innerhalb der Reaktionseinheit 4 ein Unterdruck erzeugt. Mit der Absaugvorrichtung 14-1 wird auch gezielt die Luft und damit der Sauerstoff als Bestandteil der Luft aus der Reaktionseinheit 4 abgesaugt. Innerhalb der Reaktionseinheit 4 kann derart ein Vakuum erzeugt werden.

Die über der Oberfläche des sich innerhalb des Öltanks 13 ansammelnden Öls abgesaugten Gase können zum Erzeugen von thermischer Energie und Elektroenergie direkt mit einem Blockheizkraftwerk, kurz als BHKW bezeichnet, genutzt werden.

Die Vorrichtung 1 ist zudem mit einer Steuervorrichtung 15 zum Steuern des Verfahrens zum Betreiben der Vorrichtung 1 ausgebildet. Mit der Steuervorrichtung 15 werden beispielsweise der Füllstand innerhalb des Öltanks 13, die Strömung von Öl beziehungsweise Gas und ein möglicher Defekt einer Leitung der Vorrichtung 1 bestimmt und angezeigt. Dabei ist die Steuervorrichtung 15 mit entsprechenden Sensoren verbunden. Dabei sind auch die Temperatursensoren T1, T2, T3 mit der Steuervorrichtung 15 gekoppelt. Die mit den Temperatursensoren T1, T2, T3 ermittelten Werte dienen dem Steuern der Vorrichtung 1, insbesondere des Heizsystems 2 und damit dem Beheizen der Reaktionseinheit 4 sowie der Lüfter 12-2 der Abkühlstrecke 12 und der Absaugvorrichtung 14-1. Mit der Steuervorrichtung 15 können unter anderem die Status unterschiedlicher Heizkreise des Heizsystems 2 sowie Prozesstemperaturen angezeigt werden. Ebenso kann die Anordnung des Mantelelementes 8 des Heizsystems 2 in den Zuständen geöffnet, geschlossen und teilgeöffnet ermittelt und dargestellt werden. Die Steuervorrichtung 15 dient folglich ebenso dem Ausfahren und Einfahren der Stützelemente 6 zum Öffnen und Verschließen des Heizsystems 2.

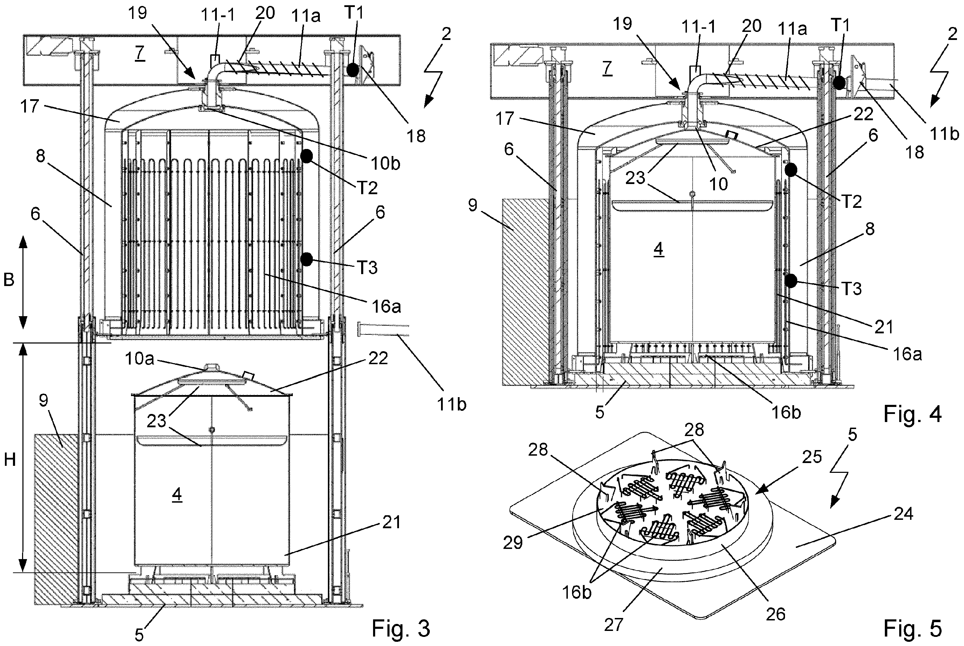

Die **Figuren 3 und 4** zeigen jeweils eine Schnittdarstellung des Heizsystems 2. In Figur 3 wird das Heizsystem 2 in geöffnetem Zustand und in Figur 4 in geschlossenem Zustand dargestellt.

Gemäß Figur 3 sind die Stützelemente 6 voll ausgefahren. Das an den oberen Enden der Stützelemente 6 angeordnete Kopfelement 7 und das mit dem Kopfelement 7 fest verbundene Mantelelement 8 sind dabei in einer Höhe H über dem Bodenelement 5 angeordnet, dass die Reaktionseinheit 4 zwischen dem Bodenelement 5 und dem Mantelelement 8 in horizontaler Richtung frei bewegbar ist.

Das Mantelelement 8 wird im unteren Bereich beweglich gegen die Stützelemente 6 abgestützt. Mittels der seitlichen Abstützung gegen die Stützelemente 6 wird eine gerade Bewegung des Mantelelementes 8 in Bewegungsrichtung B zwischen den Endpositionen gewährleistet. Eine Verkantung des Mantelelementes 8 wird vermieden.

Das Mantelelement 8 weist gleichmäßig am Umfang der inneren Fläche des Mantels verteilte Heizelemente 16a auf. Die Heizelemente 16a sind dabei im Wesentlichen in vertikaler Richtung angeordnet und werden im unteren Bereich des Mantelelementes 8 durch die Wandung zur Innenfläche geführt. Die Heizelemente 16a sind jeweils aus zwei senkrecht ausgerichteten Abschnitten ausgebildet, welche am oberen Ende mittels einer Umlenkung miteinander verbunden sind.

Das in vertikaler Richtung nach unten geöffnete Mantelelement 8 ist nach oben mit einer Haube 17 verschlossen und am Kopfelement 7 befestigt. Das Kopfelement 7 und das Mantelelement 8 bilden eine zusammenhängende Einheit. Die Haube 17 ist am Mittelpunkt mit einem Abgasstutzen 10b als Verbindung zur Abgasleitung 11a ausgebildet. Die Abgasleitung 11a erstreckt sich vom Abgasstutzen 10b durch die Haube 17 hindurch in das Kopfelement 7. Die Durchführung der Abgasleitung 11a durch die Haube 17 ist zur Haube 17 hin abgedichtet. Dabei ist die Abgasleitung 11a im Bereich des Abgasstutzens 10b mit einer in vertikaler Richtung längenveränderlichen Rohrverbindung 19, beispielsweise in Form eines Teleskoprohres, ausgebildet. Die in der Länge selbsttätig veränderliche Rohrverbindung 19 dient der Kompensation thermischer Ausdehnungen der Reaktionseinheit 4, insbesondere in Bezug auf das Mantelelement 8 mit der Haube 17 des Heizsystems 2.

Die Abgasleitung 11a ist als Übergang von der Reaktionseinheit 4 zur Destillationseinheit 3 mit einer Heizvorrichtung 20 ausgebildet. Die die Abgasleitung 11a umschließende und elektrisch betriebene Heizvorrichtung 20 ist, ebenso wie der Temperatursensor T1, mit der Steuervorrichtung 15 verbunden.

Zudem weist die Abgasleitung 11a ein Anschlusselement 11-1 zum Verbinden der Abgasleitung 11a mit einer Vorrichtung zum Einlassen eines gasförmigen Spülmediums, beispielsweise Stickstoff, auf. Das Spülmedium kann über das Anschlusselement 11-1 in die Abgasleitung 11a und insbesondere in die Reaktionseinheit 4 einströmen. Dabei ist das Anschlusselement 11-1 zwischen der Rohrverbindung 19 und dem Bereich der Abgasleitung 11a, welcher von der Heizvorrichtung 20 umschlossen ist, speziell am in vertikaler Richtung höchsten Punkt der Abgasleitung 11, 11a, angeordnet.

Die Abgasleitung 11a weist am, ausgehend vom Abgasstutzen 10b, distalen Ende ein Verbindungselement 18 auf. Das vorteilhaft als Schnellkupplung ausgebildete Verbindungselement 18 dient dabei der Verbindung der Abgasleitung 11a des Heizsystems 2 mit der Abgasleitung 11b der Destillationseinheit 3 im geschlossenen Zustand des Heizsystems 2 gemäß Figur 4. Durch die Abwärtsbewegung des Kopfelementes 7 beim Verschließen des Heizsystems 2 werden die Abgasleitungen 11a, 11b am Verbindungselement 18 sowie die Abgasstutzen 10a, 10b untereinander gekoppelt, sodass eine gasdichte Verbindung von der Reaktionseinheit 4 zur Destillationseinheit 3 hergestellt ist.

Die auf dem Bodenelement 5 angeordnete Reaktionseinheit 4 ist mit einer Wandung 21 in Form eines hohlzylindrischen Gefäßes mit einem Außendurchmesser von etwa 1,8 m ausgebildet, welche am Boden verschlossen ist. Die offene Seite der Wandung 21 ist mittels eines Deckelelementes 22 verschließbar. Zwischen der Wandung 21 und dem Deckelelement 22 ist eine Dichtung angeordnet, sodass die Reaktionseinheit 4 dicht verschlossen ist. Im Inneren der Reaktionseinheit 4 sind Siebelemente 23 ausgebildet. Die Siebelemente 23 sind dabei in horizontaler Richtung ausgerichtet und in unterschiedlichen Höhen, beabstandet zueinander angeordnet.

In der in Figur 4 gezeigten zweiten Endposition sind die Stützelemente 6 voll eingefahren. Das Mantelelement 8 sitzt auf dem Bodenelement 5 und umschließt die Reaktionseinheit 4 vollständig. Das Heizsystem 2 ist geschlossen.

Die mit Rohstoffen beschickte Reaktionseinheit 4 wird über den Boden und die Wandung 21 vorteilhaft gleichmäßig beheizt. Die Heizelemente 16a dienen dabei der Beheizung über die Wandung 21, während am Bodenelement 5 angeordnete Heizelemente 16b der Reaktionseinheit 4 Wärme durch den Boden zuführen. Die am Umfang des Mantelelementes 8 ausgebildeten Heizelemente 16a weisen im geschlossenen Zustand des Heizsystems 2 gleiche Abstände zur Wandung 21 der Reaktionseinheit 4 auf. Die Heizelemente 16a, 16b sind bevorzugt elektrisch betrieben.

Die Reaktionseinheit 4 verbleibt für eine Dauer in einem Bereich von etwa 2,5 h bis 3,5 h im Heizsystem 2, in welcher die hauptsächliche Reaktion und Umwandlung der Rohstoffe innerhalb der Reaktionseinheit 4 abläuft. Die Reaktionstemperatur innerhalb der Reaktionseinheit 4 beträgt je nach Beschickung und je nach zu erzeugenden Endprodukten zwischen 350 °C und 800 °C, insbesondere zwischen 400 °C bis 600 °C, speziell etwa 550 °C. Diese Temperatur wird mittels der zwischen der Reaktionseinheit 4 und dem Mantelelement 8 ausgebildeten Zwischenraum angeordneten Temperatursensoren T2, T3 bestimmt. Dabei wird eine Energie im Bereich von 40 kWh pro Stunde verbraucht. Die Reaktionseinheit 4 wird mit Rohstoffen einer Masse im Bereich von 2,5 t bis 3 t beschickt.

Die während des Verschwelungsprozesses entstehenden Gase werden durch den am Deckelelement 22 angeordneten Abgasstutzen 10 in die Abgasleitung 11 abgeführt, insbesondere abgesaugt. Im geschlossenen Zustand des Heizsystems 2 sind der Abgasstutzen 10a der Reaktionseinheit 4 und der Abgasstutzen 10b der Haube 17 des Mantelelementes 8 gasdicht miteinander verbunden. Damit wird sichergestellt, dass keine Gase in den Zwischenraum zwischen Reaktionseinheit 4 und Mantelelement 8 entweichen können.

Innerhalb der Reaktionseinheit 4 herrscht ein Unterdruck mit einem Absolutwert im Bereich von 2 mbar bis 10 mbar, speziell von etwa 4 mbar, welcher mit der an einem ersten Austrittsstutzen des Öltanks 13 angeordneten Absaugvorrichtung 14-1 zum Absaugen der Gase über die Oberfläche des sich innerhalb des Öltanks 13 ansammelnden Öls erzeugt wird. Mit dem gezielten Absaugen des Sauerstoffs aus der Reaktionseinheit 4 wird zum einen die Reaktionstemperatur beziehungsweise die Prozesstemperatur innerhalb der Reaktionseinheit 4 in kürzerer Zeit erreicht. Zum anderen wird damit die Strukturbildung des Kohlenstoffs als Endprodukt beeinflusst. Ein weiterer Einflussfaktor auf die Ausbildung und die Reinheit des Kohlenstoffs ist die Dauer des Verschwelungs-Prozesses. Je länger der Verschwelungs-Prozess abläuft, um so reiner ist der Kohlenstoff und kann, auch abhängig von den Ausgangsstoffen beispielsweise für medizinische Zwecke eingesetzt werden. Der für medizinische Zwecke eingesetzte Kohlenstoff ist gegebenenfalls zusätzlich zu reinigen. Während eines eher kürzeren Verschwelungs-Prozesses rückgewonnener Kohlenstoff findet zum Beispiel vorzugsweise als Filtermaterial oder im Baugewerbe Anwendung.

Zu den Einflussfaktoren auf die Ausbildung und die Reinheit des Kohlenstoffs zählt ebenso das Spülen der Reaktionseinheit 4 mit dem gasförmigen Spülmedium, insbesondere dem Stickstoff, einerseits während des Verschwelungs- und Destillationsprozesses und andererseits beim Vorgang des Abkühlens der Reaktionseinheit 4.

Mit der die Abgasleitung 11a umschließenden Heizvorrichtung 20 wird die Abgasleitung 11a erwärmt, insbesondere auf eine Temperatur im Bereich von 120 °C bis 160 °C, um derart die Temperaturdifferenz zwischen der Abgasleitung 11a und des durch die Abgasleitung 11a hindurchströmenden Abgases zu verringern. Die Temperatur des hindurchströmenden Abgases wird mittels des Temperatursensors T1 bestimmt. Die Heizvorrichtung 20 dient dabei dem Verhindern eines frühzeitigen Kondensierens des Abgases vor dem Eintritt in die Destillationseinheit 3 und damit auch einem unerwünschten Zusetzen der Abgasleitung 11a. Das Erwärmen der Abgasleitung 11a unterstützt das Ausströmen des Abgases aus der Reaktionseinheit 4.

In **Figur** 5 ist das Bodenelement 5 des Heizsystems 2 dargestellt. Das Bodenelement 5 weist eine Bodenplatte 24 und eine Zentriervorrichtung 25 für das Mantelelement 8, Heizelemente 16b sowie Stützelemente 28 zur Halterung der Reaktionseinheit 4 auf. Das Bodenelement 5 ist im Wesentlichen aus Keramik ausgebildet, um eine Wärmeisolierung nach außen, insbesondere nach unten, sicherzustellen. In Kombination mit der Wärmeisolierung des Mantelelementes 8 wird damit der Wärmeverlust des Heizsystems 2 minimiert.

Die Reaktionseinheit 4 steht auf den Stützelementen 28 der Bodenplatte 24 auf. Die Stützelemente 28 sind dabei derart ausgebildet und angeordnet, dass die Reaktionseinheit 4 bei Auflage auf den Stützelementen 28 mittig zum Bodenelement 5 ausgerichtet ist.

Die Zentriervorrichtung 25 ist in Form einer kreisrunden Scheibe mit einem Absatz ausgebildet. Die Scheibe weist folglich zwei Bereiche mit unterschiedlichen Durchmessern auf. Die zwischen den Bereichen angeordnete Kreisfläche dient als Abdichtfläche 27.

Der äußere Umfang des Bereiches der Scheibe mit dem kleineren Durchmesser ist dabei geringer als der innere Umfang der Wandung 21 der Reaktionseinheit 4 beziehungsweise des Mantelelementes 8. Im geschlossenen Zustand des Heizsystems 2 ist zwischen einer Mantelfläche 26 des Bereiches der Scheibe mit dem kleineren Durchmesser und der Innenseite der Wandung 21 ein Spalt ausgebildet. Das Mantelelement 8 steht auf der Abdichtfläche 27 der Bodenplatte 24 auf, sodass der vom Mantelelement 8 und der Bodenplatte 24 umschlossene Raum dicht verschlossen ist. Zur Abdichtung des umschlossenen Raumes sind an den korrespondieren Flächen der Bodenplatte 24 und des Mantelelementes 8 Dichtungen angeordnet. Zudem wird das Mantelelement 8 mit einem Druck im Bereich von 1 bar bis 2 bar auf die Abdichtfläche 27 der Bodenplatte 24 gedrückt und gehalten.

Da auch die Stützelemente 6 auf der Bodenplatte 24 befestigt sind, trägt die Bodenplatte 24 das gesamte Heizsystem 2.

Die Heizelemente 16b sind im Wesentlichen in horizontaler Richtung, auf einer Abschlussfläche 29 der Zentriervorrichtung 25 und senkrecht durch die Abschlussfläche 29 geführt angeordnet. Die meanderförmig gebogen ausgebildeten Heizelemente 16b weisen jeweils die Form einer Hand mit fünf Fingern auf. Die Länge der Finger nimmt dabei von außen nach innen zu, sodass der mittlere Finger die größte Länge aufweist. Die Heizelemente 16b sind symmetrisch zueinander, mit den Spitzen der Finger zum Mittelpunkt der Abschlussfläche 29 weisend, ausgerichtet.

Die Stützelemente 28, auf denen die Reaktionseinheit 4 aufsteht, ragen in vertikaler Richtung über die Heizelemente 16b hinaus, sodass der auf den Stützelementen 28 aufstehende Boden der Reaktionseinheit 4 oberhalb der Heizelemente 16b angeordnet ist. Die Heizelemente 16b weisen jeweils den gleichen Abstand zum Boden der Reaktionseinheit 4 auf, um einen gleichmäßigen Wärmeeintrag durch den Boden der Reaktionseinheit 4 zu gewährleisten.

Die Zentriervorrichtung 25, die Stützelemente 28 und die Heizelemente 16b sind konzentrisch um den Mittelpunkt der Bodenplatte 24 angeordnet.

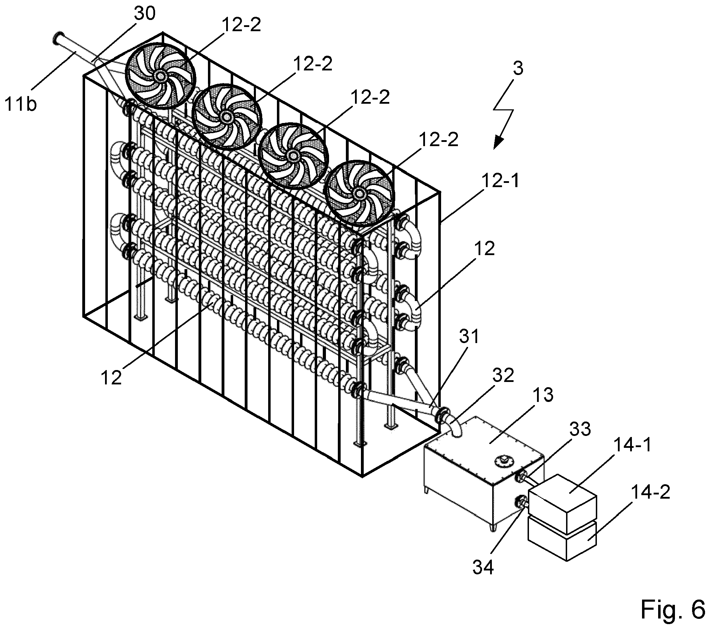

**Figur** 6 zeigt die Destillationseinheit 3, aufweisend die Abgasleitung 11b, die Abkühlstrecke 12 mit dem Luftleitgehäuse 12-1 und den Lüftern 12-2 sowie den Öltank 13 mit der Absaugvorrichtung 14-1 und der Ölfördervorrichtung 14-2 in der Reihenfolge der Durchströmungsrichtung der Endprodukte.

Die aus dem Heizsystem 2 abgeführten Gase werden durch die Abgasleitung 11b zu den ebenfalls aus Rohren ausgebildeten Abkühlstrecken 12 geleitet. Dabei wird der Gasmassenstrom an einem Abzweig 30 in zwei Teilmassenströme durch zwei parallel zueinander ausgerichtete Rohre aufgeteilt. Die Aufteilung des Gasmassenstromes bewirkt eine bessere Wärmeübertragung vom Gasmassenstrom an die Umgebung, um den Vorgang der Destillation beziehungsweise Kondensation zu optimieren. Die Rohre sind zur weiteren Verbesserung des Wärmeüberganges mit Rippen ausgebildet, um die Wärmeübertragungsflächen der Abkühlstrecken 12 zu vergrößern. Mit dem Luftleitgehäuse 12-1 und den Lüftern 12-2 wird die von den abzukühlenden Gasen abzuführende Wärmeleistung, insbesondere die Kondensationswärmemenge, weiter erhöht und gleichzeitig gesteuert. Dabei wird die Umgebungsluft als Kühlluft gleichmäßig durch das Luftleitgehäuse 12-1 hindurchgesaugt und gezielt über die Abkühlstrecken 12 geleitet. Mit der entsprechenden Leistung beziehungsweise dem Luftvolumenstrom der Lüfter 12-2 wird sichergestellt, dass die durch die Abkühlstrecke 12 der Destillationseinheit 3 hindurchströmenden Abgase bei einer Kondensationstemperatur im Bereich von 95 °C bis 125 °C verflüssigt werden können. Mit dem zusätzlichen Anströmen der Abkühlstrecken 12 werden die Abkühlstrecken 12 auf eine Temperatur unterhalb der Kondensationstemperatur der Gase abgekühlt beziehungsweise auf dem entsprechenden Temperaturniveau gehalten. Mit der derart gesteuerten Wärmeleistung wird eine höhere Ausbeute an Öl bei einer geringeren Ausbeute an Restgas erreicht. Die Temperatur wird mit dem, gemäß Figur 1, an der zwischen dem Heizsystem 2 und der Destillationseinheit 3 ausgebildeten Abgasleitung 11 angeordneten Temperatursensor T1 bestimmt.

Nach dem Durchströmen der Abkühlstrecken 12 werden die vor dem Eintritt in die Abkühlstrecken 12 aufgeteilten Teilmassenströme an einer Mündungsstelle 31 wieder vereint und durch einen Eintrittsstutzen 32 von oben in den Öltank 13 eingeleitet.

Das im Gegensatz zum Gas eine größere Dichte aufweisende Öl setzt sich im Öltank 13 ab. Der nicht kondensierbare Anteil der Destillationsprodukte wird im oberen Bereich des Öltanks 13 durch einen ersten Austrittsstutzen 33 abgeführt. Zum Absaugen der Gase über die Oberfläche des sich innerhalb des Öltanks 13 ansammelnden Öls ist nach am ersten Austrittsstutzen 33 des Öltanks 13 die Absaugvorrichtung 14-1 angeordnet. Mit dem Absaugen der Gase und dem damit erzeugten Unterdruck innerhalb der Vorrichtung 1 wird insbesondere die Luft und damit der Sauerstoff als Bestandteil der Luft aus der Reaktionseinheit 4 abgesaugt und der Verschwelungsprozess beeinflusst.

Zum Fördern des Öls aus dem Öltank 13 ist an einem zweiten Austrittsstutzen 34 des Öltanks 13 die Ölfördervorrichtung 14-2 angeordnet.

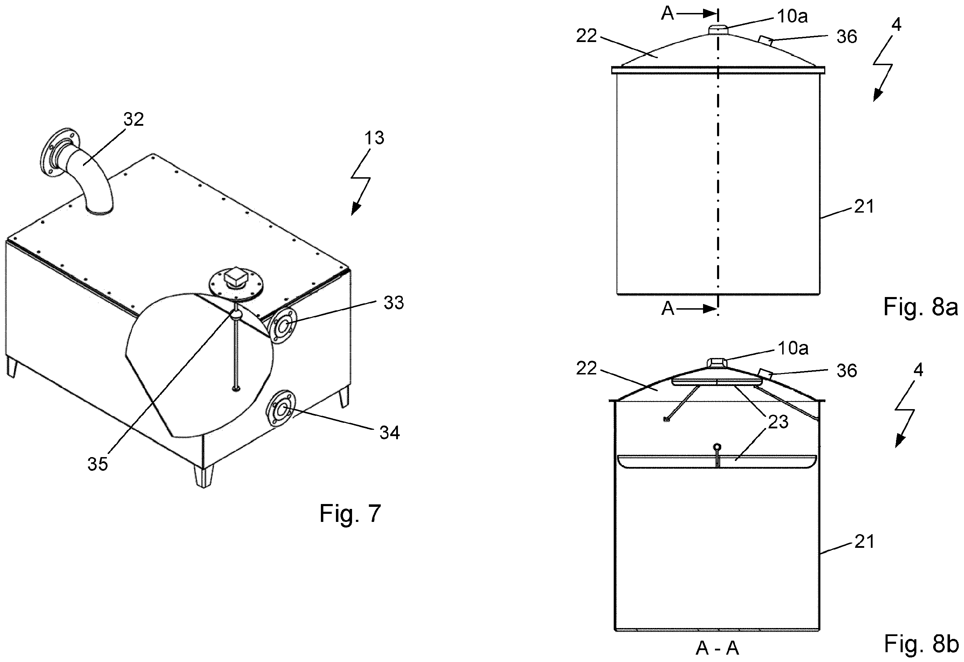

In **Figur** 7 ist ein Öltank 13 mit aufgeschnittener Seitenfläche zum Einblick in das Innere dargestellt.

Der Eintrittsstutzen 32 ist auf der Oberseite des Öltanks 13 angeordnet, sodass die Destillationsprodukte von oben in den Öltank 13 einströmen. Das Öl setzt sich am Boden des Öltanks 13 ab, während sich die im Gegensatz zum Öl geringere Dichten aufweisenden Gase oberhalb des Ölspiegels konzentrieren. Der Ölspiegel beziehungsweise Ölstand im Öltank 13 wird mit einem Schwimmer 35 bestimmt und beobachtet. Bei Erreichen einer vorgegebenen Füllhöhe wird das Öl zur weiteren Verarbeitung aus dem Öltank 13 entnommen.

Die sich im oberen Bereich des Öltanks 13 ansammelnden Gase werden durch den ersten Austrittsstutzen 33 abgeführt, insbesondere mittels der Absaugvorrichtung 14-1 abgesaugt, während das sich im unteren Bereich des Öltanks 13 ansammelnde Öl durch den zweiten Austrittsstutzen 34, insbesondere mittels der Ölfördervorrichtung 14-2, abgesaugt wird.

In den **Figuren 8a und 8b** ist jeweils die Reaktionseinheit 4 in geschlossenem Zustand dargestellt, wobei aus Figur 8b eine Schnittdarstellung der Reaktionseinheit 4 hervorgeht.

Die in Form eines hohlzylindrischen Gefäßes ausgebildete Wandung 21 mit verschlossenem Boden ist an der offenen, dem Boden gegenüberliegenden Seite mittels eines Deckelelementes 22 verschließbar. Beim Vorgang des Verschließens der Reaktionseinheit 4 wird das Deckelelement 22 in vertikaler Richtung auf die nach oben gerichtete Stirnseite der Wandung 21 aufgelegt.

Das Deckelelement 22 wird aufgrund des Eigengewichts an die Stirnseite der Wandung 21 gedrückt und liegt lösbar an der Wandung 21 an.

Zwischen der Wandung 21 und dem Deckelelement 22 ist zum dichten Verschließen der Reaktionseinheit 4 eine hochtemperaturbeständige Dichtung angeordnet. Die Reaktionseinheit 4 weist im geschlossenen Zustand eine Höhe von etwa 2,4 m auf.

Das Deckelelement 22 ist neben dem Abgasstutzen 10a mit einem Anschlussstutzen 36 ausgebildet. An dem Anschlussstutzen 36 ist eine Vorrichtung zum Einlassen eines gasförmigen Spülmediums, insbesondere von Stickstoff, in die Reaktionseinheit 4 anschließbar.

Der eigentliche Verschwelungs-Destillations-Prozess, bei welchem die Reaktionseinheit 4 innerhalb des Heizsystems 2 angeordnet ist und beheizt beziehungsweise im Wesentlichen auf der gewünschten Reaktionstemperatur gehalten wird, ist bei einer mit dem zwischen dem Heizsystem 2 und der Destillationseinheit 3 ausgebildeten Abgasleitung 11 angeordneten Temperatursensor T1 bestimmten Temperatur des Abgases von etwa 60 °C beendet. Die Reaktionseinheit 4 wird aus dem Heizsystem 2 entnommen und weist eine Temperatur beispielsweise im Bereich von 500 °C bis 600 °C auf.

Nach der Entnahme aus dem Heizsystem 2 wird die Reaktionseinheit 4 auf die in Abhängigkeit von der Produktnutzung definierte Temperatur abgekühlt. Das sich im Inneren der Reaktionseinheit 4 befindliche Gemisch wird nach dem Öffnen der Reaktionseinheit 4, das heißt nach dem Abnehmen des Deckelelementes 22, entnommen. Danach wird die Reaktionseinheit 4 wieder dem Prozess zugeführt und beschickt. Das Kohlenstoff-Eisen-Gemisch wird in seine Bestandteile getrennt.

Der rückgewonnene einzigartige Kohlenstoff wird während des Abkühlvorgangs zwischen 600 °C und 60 °C beziehungsweise 20 °C oder 30 °C innerhalb der Reaktionseinheit 4 in der sauerstofffreien Atmosphäre ohne Sauerstoff weiter gebildet. Dabei wird durch den Anschlussstutzen 36 hindurch das gasförmige Spülmedium, insbesondere Stickstoff, in die Reaktionseinheit 4 eingeströmt, was den Abkühlvorgang ebenfalls beeinflusst. Alternativ kann das gasförmige Spülmedium durch den Abgasstutzen 10a, an welchem die Vorrichtung zum Einlassen des gasförmigen Spülmediums anschließbar ist, eingeleitet werden, insbesondere wenn der Anschlussstutzen 36 nicht ausgebildet ist. Das Einströmen des Spülmediums während des Abkühlvorgangs und damit vor dem Entleeren der Reaktionseinheit 4 kann den Vorgang des Abkühlens beschleunigen, dient jedoch vor allem dem Reinigen der Endprodukte und könnte derart auch die Bildung des mit der Vorrichtung 1 rückgewonnenen Kohlenstoffs unterstützen. Mit dem Spülen der Reaktionseinheit 4 wird die Reinheit der Endprodukte, insbesondere des Kohlenstoffs, erhöht. Verunreinigungen werden ausgespült. Das durch den Anschlussstutzen 36 in die Reaktionseinheit 4 einströmende Spülmedium wird zusammen mit den Verunreinigungen durch den im Deckelelement 22 ausgebildeten Abgasstutzen 10a aus der Reaktionseinheit 4 wieder ausgelassen. Die Reaktionseinheit 4 wird bei einer Temperatur im Inneren der Reaktionseinheit 4 im Bereich von 20 °C bis 60 °C, insbesondere im Bereich von 30 °C bis 60 °C, geöffnet.

Beim Vorgang des Öffnens der Reaktionseinheit 4 wird das Deckelelement 22 in vertikaler Richtung angehoben und derart von der Reaktionseinheit 4 entfernt, sodass die Reaktionseinheit 4 entleert und anschließend wieder beschickt werden kann. Auch während des Vorgangs des Entleerens kann die Reaktionseinheit 4 mit dem Spülmedium beaufschlagt werden, um eine gewünschte Reinheit der Endprodukte, insbesondere des Kohlenstoffs, zu erreichen. Der Kohlenstoff wird beim Entleeren der Reaktionseinheit 4 vorzugsweise abgesaugt.

Am Verschwelungs-Destillations-Prozess zur stofflichen Behandlung der Rohstoffe sind gleichzeitig vier aus hochtemperaturbeständigem Stahl gefertigte Reaktionseinheiten 4 mit jeweils einer Füllmenge im Bereich von 2,5 t bis 3,5 t (75 % mechanisch, 25 % automatisiert) beteiligt. Während die erste Reaktionseinheit 4 beschickt wird, wird die zweite Reaktionseinheit 4, welche bereits beschickt ist, vorgewärmt. Die dritte Reaktionseinheit 4 ist währenddessen bereits dem Heizsystem 2 zugeführt und wird beheizt, sodass der eigentliche Verschwelungs-Destillations-Prozess abläuft. Die vierte Reaktionseinheit 4 wird unterdessen abgekühlt und anschließend entleert. Durch die Anwendung des modularen Systems, beispielsweise mit vier Reaktionseinheiten 4, kann schrittweise der Durchsatz erhöht und an den jeweiligen Bedarf flexibel angepasst werden. Der gesamte Prozess läuft quasikontinuierlich ab.

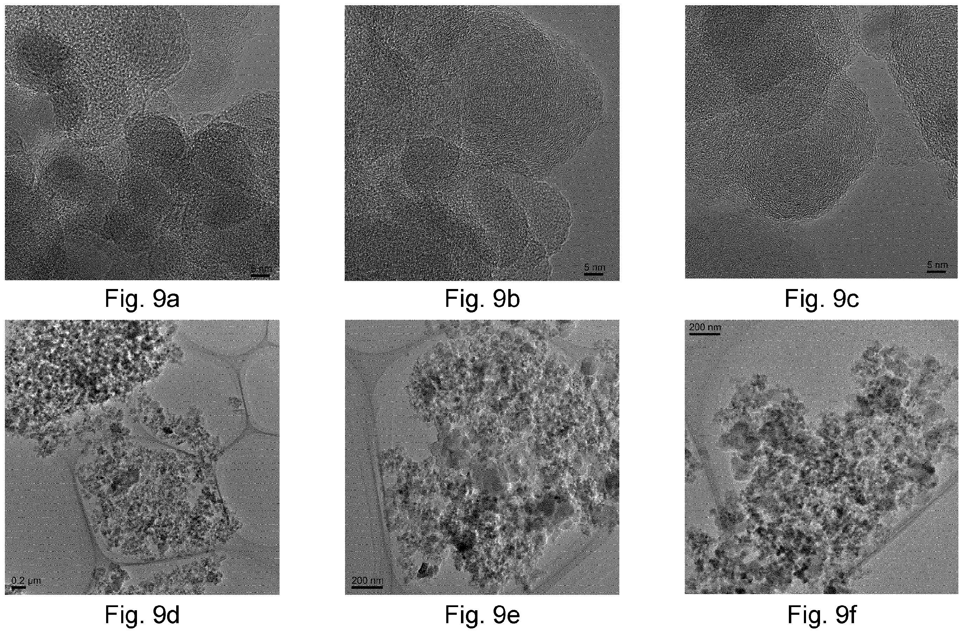

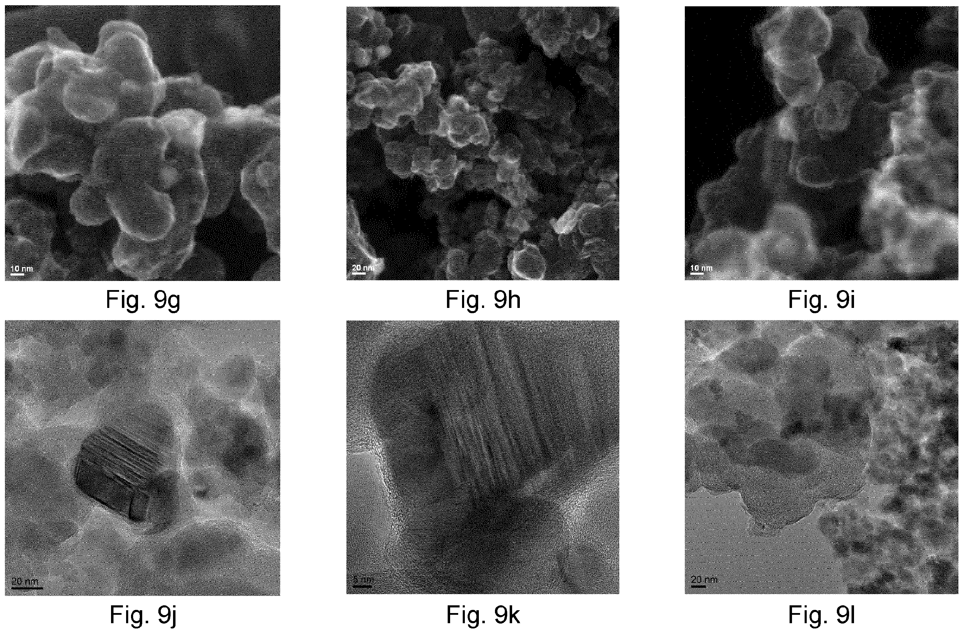

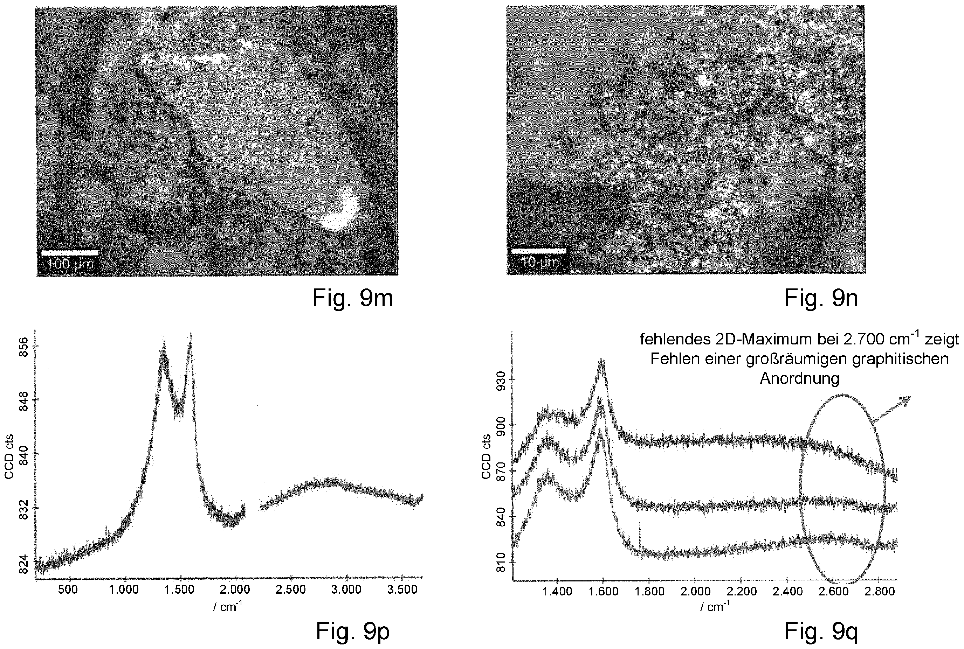

In den **Figuren 9a bis 9n** sind mikroskopische Aufnahmen von mit der Vorrichtung 1 zur stofflichen Behandlung von Rohstoffen erzeugtem Kohlenstoff gezeigt. Aus den mit einem Transmissionselektronenmikroskop, kurz als TEM bezeichnet, erstellten Aufnahmen ist eine Struktur des Kohlenstoffs erkennbar. Die Transmissionselektronenmikroskopie dient dem Erkennen und Charakterisieren von Struktur und Partikelgröße von Stoffen und Stoffgemischen im Nanometer-Bereich.

Die Aufnahmen zeigen eine sehr feinteilige, dreidimensionale, homogene und pseudokristalline Struktur der primären Kohlenstoffpartikel im Subnanometer-Bereich mit einer sehr großen inneren Oberfläche. Die Kohlenstoffpartikel sind partiell als größere Agglomerate mit gleicher Oberflächenstruktur zu erkennen.

Aus den **Figuren 9p und 9q** gehen Ergebnisse einer Raman-Spektroskopie des Kohlenstoffs hervor. Das fehlende 2D-Maximum bei 2.700 cm-1 zeigt das Fehlen einer großräumigen graphitischen Anordnung. Bei dem mit der Vorrichtung 1 zur stofflichen Behandlung von Rohstoffen erzeugten Kohlenstoff handelt es sich um amorphen Kohlenstoff, in welchem die Nanoteilchen ohne langreichweitige Ordnung vernetzt sind. Der Kohlenstoff weist weder Nanotubes noch eine strukturelle Ähnlichkeit mit Graphen auf.

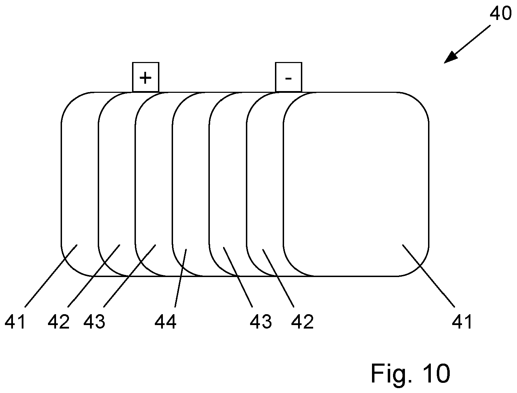

In **Figur** 10 ist ein elektrischer Energiespeicher als Speichervorrichtung 40 in Form eines Superkondensators in Schichtbauweise dargestellt. Der Superkondensator kann als Speichervorrichtung 40 eine symmetrische Bauform mit einer Doppelschicht des Kohlenstoffs aufweisen. Dabei sind der Aufbau des negativen und des positiven Pols identisch. Die Pole können beim ersten Ladeprozess definiert werden.

Die Speichervorrichtung 40 weist von außen nach innen jeweils ein Gehäuse 41 und einen Kollektor 42 mit einer als Kohlenstoffschicht ausgebildeten Elektrode 43 auf. Im Inneren sind ein Separator 44 und ein Elektrolyt vorgesehen. Von innen nach außen sind an den Separator 44 mit dem Elektrolyt folglich beiderseits jeweils die als Kohlenstoffschicht ausgebildete Elektrode 43, ein Kollektor 42 und das Gehäuse 41 angeordnet. Die Speichervorrichtung 40 weist folglich eine Doppelschicht auf.

Das Gehäuse 41 kann als eine Laminierfolie aus Polyester, kurz als PET bezeichnet, ausgebildet sein, welche an einer nach innen ausgerichteten Seite eine Klebeschicht aus Ethyl-Vinyl-Acetat, kurz als EVA bezeichnet, aufweist. Mit dem Gehäuse 41 werden die Kollektoren 42 mit den Elektroden 43 zusammengedrückt. Das Innere der Speichervorrichtung 40 wird frei von Sauerstoff gehalten, um eine Oxydation zu verhindern.

An den jeweils vorzugsweise aus einer Messingfolie mit einer Stärke von etwa 0,2 mm und einer Oberfläche von 10 cm² ausgebildeten Kollektoren 42 werden die Ionen angelagert. Die Kollektoren 42 stellen die elektrische Verbindung für den elektrischen Strom von den Anschlüssen der Speichervorrichtung 40 zu den Elektroden 43 her. Die Kollektoren 42, insbesondere die Elektroden 43, sind mit dem Elektrolyten kompatibel, sodass eine unerwünschte Reaktion vermieden wird.

Der zerkleinerte, insbesondere vorab mit einem Mixer bearbeitete und anschließend mit einem Mörser weiter verfeinerte Kohlenstoff wird mit Methylalkohol, speziell mit 99 %-igem Methylalkohol, gemischt und auf die angeschliffene und anschließend entfettete Messingfolie aufgetragen. Dabei kann die Mischung aus Kohlenstoff und Methylalkohol mit einem Spritzwerkzeug, insbesondere einer Luftspritzpistole, auch als "Airbrush-Pistole" bezeichnet, aufgetragen werden. Da der Methylalkohol nach dem Auftragen auf die Messingfolie vollkommen verdunstet, ist das Mischverhältnis aus Kohlenstoff und Methylalkohol irrelevant und kann an das Spritzwerkzeug angepasst werden. Mit dem Spritzwerkzeug können mehrere Lagen, vorzugsweise eine erste und eine zweite Lage, aufgetragen werden.

Für eine weitere Schicht wird Natronwasserglas, insbesondere 10 %-iges Natronwasserglas, mit demineralisiertem Wasser im Verhältnis 1:1 gemischt. Anschließend wird der Mischung aus Natronwasserglas und demineralisiertem Wasser Kohlenstoff hinzugefügt. Dabei werden zu 40 ml Lösung 10 ml Kohlenstoff, welcher als flockiges Volumen bestimmt wird, hinzugegeben. Das fertige Gemisch wird ebenfalls mittels des Spritzwerkzeugs vorzugsweise jeweils in zwei Lagen auf die Messingfolie, insbesondere die bereits vorhandenen Lagen Kohlenstoff, aufgetragen. Damit werden die unteren Lagen gebunden, welche ein Teil der Flüssigkeit absorbieren.

Mit dem durch das Verfahren zur stofflichen Behandlung von Rohstoffen hergestellten Kohlenstoff mit der Struktur der dreidimensionalen Anordnung von Kohlenstoffnanoteilchen und sehr großer Oberfläche wird eine maximale Kapazität bei minimalem Volumen und Gewicht erreicht, da insbesondere die Oberfläche der Elektroden 43 den Wert der Kapazität bestimmt. Die aus dem Kohlenstoff ausgebildeten Elektroden 43 sind zudem gegenüber dem Elektrolyten chemisch inert und weisen eine hohe Temperaturstabilität auf.

Als Elektrolyt kann Phosphorsäure, kurz H₃PO₄, gesättigt mit Natriumhydroxid, kurz NaOH, verwendet werden. Zudem wird zusätzlich Natriumhydroxid dazu gegeben. Dabei werden 10 ml Phosphorsäure sechs Perlen Natriumhydroxid hinzugefügt, um die elektrische Leitfähigkeit zu erhöhen.

Der dem Verhindern eines Kurzschlusses innerhalb der Speichervorrichtung 40 dienende Separator 44 wird mit dem Elektrolyten getränkt. Die Stärke und die Dichte des Separators 44 bestimmen die Spannung und die automatische Entladung des Superkondensators. Als Separator 44 kann eine fusselfreie Papierschicht in Form reiner Cellulose mit einer Stärke von etwa 0,1 mm dienen. Die Papierschicht kann als eine doppelte Schicht ausgebildet sein.

Die derartig erzeugte Speichervorrichtung 40 kann als Energiezelle mit 0,5 V geladen werden. Dabei wird die Energie ohne Temperaturänderung in kürzester Zeit aufgenommen und kann auch ebenso ohne Temperaturänderung schnell wieder abgegeben werden. Mit den Eigenschaften des Superkondensators kann eine Spannung von 0,5 V bis über 10 V erreicht werden. Die Speichervorrichtung 40 ist zu verschiedenen Größen skalierbar. Ein elektrischer Energiespeicher kann aus einer Vielzahl an Speichervorrichtungen 40 ausgebildet sein.

Eine Speichervorrichtung 40 mit einem Gesamtgewicht von 2 g enthält 0,3 g Kohlenstoff und ist mit einer Spannung von 5,24 V innerhalb einer Zeitspanne von 1 s vollständig geladen. Die Speichervorrichtung 40 weist eine elektrische Kapazität von 140 mF auf. Die Stromstärke beträgt folglich 0,73 A. Die derartige Speichervorrichtung 40 ist in der Größe skalierbar.

Bei einem Vergleich mit herkömmlichen Materialien für die Herstellung von Superkondensatoren mit Aktivkohle aus Kokosnussfasern als Elektroden und ansonsten gleichen Parametern und Abmessungen beträgt die elektrische Kapazität lediglich 71 mF. Die mit der Aktivkohle hergestellte Speichervorrichtung entlädt sich zudem um ein Vielfaches schneller. Die Unterschiede sind vor allem auf die unterschiedlichen Werte der BET-Oberfläche der Aktivkohle im Vergleich zum durch das Verfahren zur stofflichen Behandlung von Rohstoffen hergestellten, amorphen Kohlenstoffs mit der Struktur der dreidimensionalen Anordnung von Kohlenstoffnanoteilchen zurückzuführen. Der amorphe Kohlenstoff weist zudem eine um ein Vielfaches höhere Hitzebeständigkeit auf.

Nach alternativen Ausführungsformen sind die Kollektoren 42 aus einer Graphitfolie, welche weniger anfällig für Säure ist und entsprechend eine höhere Flexibilität in der Wahl des Elektrolyten zulässt, und einer Aluminiumfolie ausgebildet.

Für ein Erhöhen der möglichen Spannung der Speichervorrichtung 40 kann der Kohlenstoff vor der Verarbeitung insbesondere in einem Säurebad gereinigt beziehungsweise zum Aktivieren, beispielsweise in einer Mikrowelle, auf bis zu 800 °C erhitzt werden. Anstelle des Natronwasserglases können Polyurethan, Casein oder Aceton mit Weißleim als Bindemittel verwendet werden. Die Elektroden 43 können jeweils in gefalteter Form vorliegen, um die Auflagefläche zu erhöhen.

Als Elektrolyt werden auf Wasser basierende Lösungen, wie Zink- und Sodium-Sulfate, sowie organische Lösungen, wie Ethyl-Acetat, verwendet. Der Elektrolyt kann auf Wasserbasis mit Natriumsulfat, kurz Na₂SO₄, hergestellt und vorzugsweise, beispielsweise in einer Mikrowelle, aktiviert sein. Als Elektrolyt kann ebenso ein Redox-Elektrolyt verwendet werden.

Als Separator 44 ist ein synthetisches Material, insbesondere eine dünne Polypropylenmembran mit feinsten Poren oder ein Glasfasergewebe, nutzbar.

### LISTE DER BEZUGSZEICHEN

- 1: Vorrichtung zur stofflichen Behandlung
- 2: Heizsystem
- 3: Destillationseinheit
- 4: Reaktionseinheit
- 5: Bodenelement des Heizsystems 2
- 6: Stützelement
- 7: Kopfelement des Heizsystems 2
- 8: Mantelelement des Heizsystems 2
- 9: Umhausung
- 10, 10a, 10b: Abgasstutzen
- 11, 11a, 11b: Abgasleitung
- 11-1: Anschlusselement der Abgasleitung 11, 11a
- 12: Abkühlstrecke der Destillationseinheit 3
- 12-1: Luftleitgehäuse
- 12-2: Lüfter
- 13: Öltank
- 14-1: Absaugvorrichtung
- 14-2: Ölfördervorrichtung
- 15: Steuervorrichtung
- 16a, 16b: Heizelement
- 17: Haube
- 18: Verbindungselement der Abgasleitung 11, 11a
- 19: Rohrverbindung
- 20: Heizvorrichtung
- 21: Wandung der Reaktionseinheit 4
- 22: Deckelelement
- 23: Siebelement
- 24: Bodenplatte
- 25: Zentriervorrichtung für Mantelelement 8
- 26: Mantelfläche der Zentriervorrichtung 25
- 27: Abdichtfläche der Zentriervorrichtung 25
- 28: Stützelement für Reaktionseinheit 4
- 29: Abschlussfläche
- 30: Abzweig
- 31: Mündungsstelle
- 32: Eintrittsstutzen des Öltanks 13
- 33: erster Austrittsstutzen des Öltanks 13
- 34: zweiter Austrittsstutzen des Öltanks 13
- 35: Schwimmer
- 36: Anschlussstutzen des Deckelelements 22

- 40: Speichervorrichtung
- 41: Gehäuse
- 42: Kollektor
- 43: Elektrode
- 44: Separator

- B: Bewegungsrichtung des Heizsystems 2
- H: Höhe

- T1, T2, T3: Temperatursensor

## Patentansprüche

1. Kohlenstoff mit einer Struktur einer dreidimensionalen Anordnung von Kohlenstoffnanoteilchen hergestellt durch ein Verfahren zur stofflichen Behandlung von Rohstoffen, welches folgende Schritte aufweist:
- Erwärmen einer mit Rohstoffen beschickten und in einem geschlossenen Heizsystem (2) angeordneten Reaktionseinheit (4) sowie Starten eines Verschwelungs- und Destillationsprozesses, wobei der Verschwelungs- und Destillationsprozess durch gezieltes Beheizen bei einer im Wesentlichen konstanten Temperatur innerhalb der Reaktionseinheit (4) erfolgt,
- Abführen entstehender Gase aus der Reaktionseinheit (4) in eine Destillationseinheit (3) durch eine zwischen der Reaktionseinheit (4) und der Destillationseinheit (3) ausgebildete Abgasleitung (11, 11a) sowie Bestimmen der Temperatur des durch die Abgasleitung (11, 11a) hindurchströmenden Gases,
- Abkühlen und Kondensieren der Gase in der Destillationseinheit (3), wobei durch eine Zwangskühlung einer Abkühlstrecke (12) der Destillationseinheit (3) die Temperatur der Gase über eine von den Gasen abgeführte Wärmeleistung gesteuert wird, und
- Absaugen nichtkondensierbarer Gase, wobei innerhalb der Reaktionseinheit (4) ein Unterdruck zur Umgebung erzeugt und Sauerstoff aus der Reaktionseinheit (4) abgeführt wird,
zur medizinischen Verwendung.

2. Kohlenstoff zur medizinischen Verwendung nach Anspruch 1, **dadurch gekennzeichnet, dass** die Verwendung zur Hämoperfusion/-adsorption dient.

3. Kohlenstoff zur medizinischen Verwendung nach Anspruch 1, **dadurch gekennzeichnet, dass** die Verwendung zur topischen Anwendung auf der Haut, insbesondere zur Wundheilung, speziell zur Behandlung von Wunden und Wundoberflächen, dient.

4. Kohlenstoff zur medizinischen Verwendung nach Anspruch 1, **dadurch gekennzeichnet, dass** die Verwendung zur Applikation bei Vergiftungen, insbesondere zur oralen Aufnahme als Antidot, dient.

5. Kohlenstoff zur medizinischen Verwendung nach Anspruch 1, **dadurch gekennzeichnet, dass** der Kohlenstoff als ein Trägermolekül, insbesondere für Antibiotika, dient.

6. Kohlenstoff zur medizinischen Verwendung nach Anspruch 1, **dadurch gekennzeichnet, dass** die Verwendung zum Beschichten, insbesondere für Implantate, dient.

7. Verwendung eines Kohlenstoffs mit einer Struktur einer dreidimensionalen Anordnung von Kohlenstoffnanoteilchen hergestellt durch ein Verfahren zur stofflichen Behandlung von Rohstoffen, welches folgende Schritte aufweist:
- Erwärmen einer mit Rohstoffen beschickten und in einem geschlossenen Heizsystem (2) angeordneten Reaktionseinheit (4) sowie Starten eines Verschwelungs- und Destillationsprozesses, wobei der Verschwelungs- und Destillationsprozess durch gezieltes Beheizen bei einer im Wesentlichen konstanten Temperatur innerhalb der Reaktionseinheit (4) erfolgt,
- Abführen entstehender Gase aus der Reaktionseinheit (4) in eine Destillationseinheit (3) durch eine zwischen der Reaktionseinheit (4) und der Destillationseinheit (3) ausgebildete Abgasleitung (11, 11a) sowie Bestimmen der Temperatur des durch die Abgasleitung (11, 11a) hindurchströmenden Gases,
- Abkühlen und Kondensieren der Gase in der Destillationseinheit (3), wobei durch eine Zwangskühlung einer Abkühlstrecke (12) der Destillationseinheit (3) die Temperatur der Gase über eine von den Gasen abgeführte Wärmeleistung gesteuert wird, und
- Absaugen nichtkondensierbarer Gase, wobei innerhalb der Reaktionseinheit (4) ein Unterdruck zur Umgebung erzeugt und Sauerstoff aus der Reaktionseinheit (4) abgeführt wird,
als thermischer und/oder feuerfester und/oder strahlungsresistenter Isolierstoff.

8. Verwendung des Kohlenstoffs nach Anspruch 7, **dadurch gekennzeichnet, dass** der Isolierstoff als ein Bestandteil eines Hitzeschildes bei Raketen und/oder Raumgleitern ausgebildet ist.

9. Verwendung des Kohlenstoffs nach Anspruch 7, **dadurch gekennzeichnet, dass** der Isolierstoff als ein Bestandteil einer Umhausung, insbesondere eines Kraftwerks oder von Nuklearreaktoren, ausgebildet ist.

10. Verwendung des Kohlenstoffs nach Anspruch 7, **dadurch gekennzeichnet, dass** der Isolierstoff als ein Bestandteil einer Wandung eines Hauses ausgebildet ist.

11. Verwendung eines Kohlenstoffs mit einer Struktur einer dreidimensionalen Anordnung von Kohlenstoffnanoteilchen hergestellt durch ein Verfahren zur stofflichen Behandlung von Rohstoffen, welches folgende Schritte aufweist:
- Erwärmen einer mit Rohstoffen beschickten und in einem geschlossenen Heizsystem (2) angeordneten Reaktionseinheit (4) sowie Starten eines Verschwelungs- und Destillationsprozesses, wobei der Verschwelungs- und Destillationsprozess durch gezieltes Beheizen bei einer im Wesentlichen konstanten Temperatur innerhalb der Reaktionseinheit (4) erfolgt,
- Abführen entstehender Gase aus der Reaktionseinheit (4) in eine Destillationseinheit (3) durch eine zwischen der Reaktionseinheit (4) und der Destillationseinheit (3) ausgebildete Abgasleitung (11, 11a) sowie Bestimmen der Temperatur des durch die Abgasleitung (11, 11a) hindurchströmenden Gases,
- Abkühlen und Kondensieren der Gase in der Destillationseinheit (3), wobei durch eine Zwangskühlung einer Abkühlstrecke (12) der Destillationseinheit (3) die Temperatur der Gase über eine von den Gasen abgeführte Wärmeleistung gesteuert wird, und
- Absaugen nichtkondensierbarer Gase, wobei innerhalb der Reaktionseinheit (4) ein Unterdruck zur Umgebung erzeugt und Sauerstoff aus der Reaktionseinheit (4) abgeführt wird,
als ein Filterelement.

12. Verwendung des Kohlenstoffs nach Anspruch 11, **dadurch gekennzeichnet, dass** das Filterelement als ein Wasserfilterelement oder als ein Luftfilterelement ausgebildet ist.

13. Verwendung des Kohlenstoffs nach Anspruch 11 oder 12, **dadurch gekennzeichnet, dass** die Verwendung zum Ausfiltern von Salz, Öl, Benzin, Iod oder Säure aus Wasser dient.

14. Verwendung des Kohlenstoffs nach Anspruch 11 oder 12, **dadurch gekennzeichnet, dass** das Filterelement, insbesondere das Luftfilterelement, als ein Bestandteil einer Klimaanlage oder einer Atemschutzmaske oder einer Abgasvorrichtung ausgebildet ist.

15. Verwendung eines Kohlenstoffs mit einer Struktur einer dreidimensionalen Anordnung von Kohlenstoffnanoteilchen hergestellt durch ein Verfahren zur stofflichen Behandlung von Rohstoffen, welches folgende Schritte aufweist:
- Erwärmen einer mit Rohstoffen beschickten und in einem geschlossenen Heizsystem (2) angeordneten Reaktionseinheit (4) sowie Starten eines Verschwelungs- und Destillationsprozesses, wobei der Verschwelungs- und Destillationsprozess durch gezieltes Beheizen bei einer im Wesentlichen konstanten Temperatur innerhalb der Reaktionseinheit (4) erfolgt,
- Abführen entstehender Gase aus der Reaktionseinheit (4) in eine Destillationseinheit (3) durch eine zwischen der Reaktionseinheit (4) und der Destillationseinheit (3) ausgebildete Abgasleitung (11, 11a) sowie Bestimmen der Temperatur des durch die Abgasleitung (11, 11a) hindurchströmenden Gases,
- Abkühlen und Kondensieren der Gase in der Destillationseinheit (3), wobei durch eine Zwangskühlung einer Abkühlstrecke (12) der Destillationseinheit (3) die Temperatur der Gase über eine von den Gasen abgeführte Wärmeleistung gesteuert wird, und
- Absaugen nichtkondensierbarer Gase, wobei innerhalb der Reaktionseinheit (4) ein Unterdruck zur Umgebung erzeugt und Sauerstoff aus der Reaktionseinheit (4) abgeführt wird,
als ein Speicherelement.

16. Verwendung des Kohlenstoffs nach Anspruch 15, **dadurch gekennzeichnet, dass** das Speicherelement als eine Komponente eines elektrischen Energiespeichers ausgebildet ist.

17. Verwendung des Kohlenstoffs nach Anspruch 15 oder 16, **dadurch gekennzeichnet, dass** das Speicherelement als eine Komponente einer Batterie, insbesondere einer Batterie eines Kraftfahrzeugs, ausgebildet ist.

18. Verwendung des Kohlenstoffs nach Anspruch 15 oder 16, **dadurch gekennzeichnet, dass** das Speicherelement als eine Komponente eines Kondensators ausgebildet ist.

19. Verwendung des Kohlenstoffs nach Anspruch 15, **dadurch gekennzeichnet, dass** das Speicherelement als eine Komponente eines Datenspeichers ausgebildet ist.

20. Speichervorrichtung (40) als elektrischer Energiespeicher in Form eines Doppelschicht-Kondensators, aufweisend einen symmetrischen Aufbau mit von außen nach innen jeweils einem Gehäuse (41) und einem Kollektor (42) mit einer als Kohlenstoffschicht ausgebildeten Elektrode (43) und einem Separator (44) mit einem Elektrolyten, **dadurch gekennzeichnet, dass** die Kohlenstoffschicht aus einem mit einem Verfahren zur stofflichen Behandlung von Rohstoffen, aufweisend folgende Schritte:
- Erwärmen einer mit Rohstoffen beschickten und in einem geschlossenen Heizsystem (2) angeordneten Reaktionseinheit (4) sowie Starten eines Verschwelungs- und Destillationsprozesses, wobei der Verschwelungs- und Destillationsprozess durch gezieltes Beheizen bei einer im Wesentlichen konstanten Temperatur innerhalb der Reaktionseinheit (4) erfolgt,
- Abführen entstehender Gase aus der Reaktionseinheit (4) in eine Destillationseinheit (3) durch eine zwischen der Reaktionseinheit (4) und der Destillationseinheit (3) ausgebildete Abgasleitung (11, 11a) sowie Bestimmen der Temperatur des durch die Abgasleitung (11, 11a) hindurchströmenden Gases,
- Abkühlen und Kondensieren der Gase in der Destillationseinheit (3), wobei durch eine Zwangskühlung einer Abkühlstrecke (12) der Destillationseinheit (3) die Temperatur der Gase über eine von den Gasen abgeführte Wärmeleistung gesteuert wird, und
- Absaugen nichtkondensierbarer Gase, wobei innerhalb der Reaktionseinheit (4) ein Unterdruck zur Umgebung erzeugt und Sauerstoff aus der Reaktionseinheit (4) abgeführt wird,
hergestellten Kohlenstoff mit einer Struktur einer dreidimensionalen Anordnung von Kohlenstoffnanoteilchen ausgebildet ist.

21. Verwendung eines Kohlenstoffs mit einer Struktur einer dreidimensionalen Anordnung von Kohlenstoffnanoteilchen hergestellt durch ein Verfahren zur stofflichen Behandlung von Rohstoffen, welches folgende Schritte aufweist:
- Erwärmen einer mit Rohstoffen beschickten und in einem geschlossenen Heizsystem (2) angeordneten Reaktionseinheit (4) sowie Starten eines Verschwelungs- und Destillationsprozesses, wobei der Verschwelungs- und Destillationsprozess durch gezieltes Beheizen bei einer im Wesentlichen konstanten Temperatur innerhalb der Reaktionseinheit (4) erfolgt,
- Abführen entstehender Gase aus der Reaktionseinheit (4) in eine Destillationseinheit (3) durch eine zwischen der Reaktionseinheit (4) und der Destillationseinheit (3) ausgebildete Abgasleitung (11, 11a) sowie Bestimmen der Temperatur des durch die Abgasleitung (11, 11a) hindurchströmenden Gases,
- Abkühlen und Kondensieren der Gase in der Destillationseinheit (3), wobei durch eine Zwangskühlung einer Abkühlstrecke (12) der Destillationseinheit (3) die Temperatur der Gase über eine von den Gasen abgeführte Wärmeleistung gesteuert wird, und
- Absaugen nichtkondensierbarer Gase, wobei innerhalb der Reaktionseinheit (4) ein Unterdruck zur Umgebung erzeugt und Sauerstoff aus der Reaktionseinheit (4) abgeführt wird,
zum Herstellen von pflanzlichen Produkten oder zum Bepflanzen von wasserarmen Gebieten, wobei der Kohlenstoff als Wasserspeicher und Nährstoffspeicher, insbesondere zum Anreichern oberer Bodenschichten, dient.

22. Verwendung des Kohlenstoffs nach Anspruch 21, **dadurch gekennzeichnet, dass** die Verwendung dem Herstellen von pflanzlichen Nahrungsmitteln dient.

23. Verwendung des Kohlenstoffs nach Anspruch 21 oder 22, **dadurch gekennzeichnet, dass** die Verwendung als natürlicher Halmstabilisator in der Getreideproduktion dient.

24. Verwendung des Kohlenstoffs nach einem der Ansprüche 21 bis 23, **dadurch gekennzeichnet, dass** der Kohlenstoff in einer oder mehreren Schichten großflächig in den Boden, insbesondere in einer Tiefe von etwa 20 cm bis 30 cm, eingebracht wird.
